# EUROPEAN PATENT SPECIFICATION

(11) **EP 1 127 168 B1**
(45) Date of publication and mention of the grant of the patent: **06.08.2008**
(21) Application number: 99972008.9
(22) Date of filing: 01.11.1999
(51) Int. Cl.: C12Q 1/68, A61K 39/00, A61K 31/70, A61P 43/00

(54) **METHOD FOR DETERMINING THE SUSCEPTIBILITY TO DEVELOPING SEPSIS AND THERAPEUTICS FOR THE TREATMENT OF SEPSIS**
VERFAHREN ZUR BESTIMMUNG DER SUZEPTIBILITÄT ZUR SEPSISENTWICKLUNG UND ARZNEIMITTEL ZUR BEHANDLUNG VON SEPSIS
DIAGNOSTIC ET TRAITEMENT D'UNE SEPTICEMIE

(30) Priority: 30.10.1998 US 183850
(43) Date of publication of application: 29.08.2001
(73) Proprietor: Interleukin Genetics, Inc., Waltham, MA 02452 (US)
(72) Inventor: DI GIOVINE, Francesco, S., Sheffield S10 3GZ (GB); DUFF, Gordon, W., Sheffield S10 3BZ (GB)
(74) Representative: Crump, Julian Richard John
(86) International application number: PCT/US1999/025633
(87) International publication number: WO 2000/037679

(56) References cited:
- EP-A- 0 769 560
- WO-A-95/01997
- WO-A-97/34616
- WO-A-98/15653
- WO-A-98/40517
- WO-A-98/54359
- WO-A-99/24615
- FANG, XIANGMING ET AL: "Genomic polymorphisms of the interleukin-1 gene family in patients suffering from severe sepsis." ANESTHESIOLOGY (HAGERSTOWN), (1997) VOL. 87, NO. 3 SUPPL., PP. A264. MEETING INFO.: ANNUAL MEETING OF THE AMERICAN SOCIETY OF ANESTHESIOLOGIST SAN DIEGO, CALIFORNIA, USA OCTOBER 18-22, 1997 , XP000938923
- FANG, X. M. ET AL: "IL-1RA genomic polymorphism associated with susceptibility to and outcome of severe sepsis." ACTA ANAESTHESIOLOGICA SCANDINAVICA, (1996) VOL. 40, NO. SUPPL. 109, PP. 236. MEETING INFO.: THE VIENNA INTERNATIONAL ANESTHESIOLOGY AND INTENSIVE CARE CONGRESS VIENNA, AUSTRIA OCTOBER 2-5, 1996 , XP000938930
- COX A ET AL: "AN analysis of linkage disequilibrium in the interleukin-1 gene cluster, using a novel grouping method for multiallelic markers" AMERICAN JOURNAL OF HUMAN GENETICS, May 1998 (1998-05), pages 1180-1188, XP002077316
- CLAY ET AL: "Novel interleukin-1 receptor antagonist polymorphisms and their use in allele-specific mRNA assessment" HUMAN GENETICS, vol. 97, 1996, pages 723-726, XP000938836 cited in the application
- FANG X M ET AL: "Comparison of two polymorphisms of the interleukin-1 gene family: interleukin-1 receptor antagonist polymorphism contributes to susceptibility to severe sepsis [see comments]." CRITICAL CARE MEDICINE, (1999 JUL) 27 (7) 1330-4. , XP000938972

## Description

### 1. BACKGROUND OF THE INVENTION

### Sepsis

Clinical infection is the biological end result of a number of factors, including the nature of the invading organism, its intrinsic virulence, the microenvironment of the invaded tissue or organ, and the responsiveness of the host. Any means by which bacteria can be introduced into the tissues can result in an infection. However, the nature of the introduction can influence the severity of the infection and can alter the host's ability to respond. As injuries, a cutaneous laceration, for example, differs from an extensive surgical dissection, which in turn differs from a perforated gastrointestinal viscus. Similarly, a lung infection (a pneumonia) occurring in an area of atelectasis is different from a lung infection that takes place as a result of an aspiration event. Mere presence of pathogens in intact or injured areas does not comprise an infection. A certain critical mass of organisms is necessary in order to sufficiently overcome the host defenses and cause an invasive infection. This level of bacteria is usually stated to be 10⁵ organisms per gram of treatment. A variety of factors can influence the balance between microbial invader and host defenses sufficiently that infections develop at lower levels of bacterial exposure. Necrotic tissue or foreign bodies in a wound are termed adjuvant factors, understood to make infections likely to develop at lower concentrations. Local physiological factors such as impaired circulation also increase local susceptibility to infection. Systemic ailments like diabetes, uremia and AIDS are known to lower the host's resistance to infection, again making it easier for microbes to establish an infection in the tissues.

The severity of an infection in part relates to the extent of the injury that accompanies or precedes it. More severe injury (e.g., an extensive accidental or surgical trauma) interferes with host integrity more substantially, permitting freer access to host tissues and compromising intrinsic host defenses. The severity of an infection depends upon the number and kind of micro-organisms responsible for the infection. If a polymicrobial infection is diagnosed or suspected, early and aggressive antibiotic intervention is commonly warranted, often with broad-spectrum agents with activity against a number of possible invaders.

Certain virulence factors have been associated with specific microorganisms, making invasion carried out by these cells more destructive. Virulence factors are of three general types: 1) biological products produced and secreted by the infecting agent that attack cells in the host or that affect host homeostatic mechanisms to produce clinical disease; 2) structural components of the normal bacterial cell which, when shed within the host's internal environment or when released following death and lysis of the bacterial cell, have toxic effects on the host; 3) responses of the microorganism to antibiotics that make them resistant to these chemotherapeutic agents. Particular microorganisms characteristically manifest specific virulence factors. For example, *Staphylococcus aureus* produces coagulase, which acts as a powerful virulence factor. *Staph*. and *Streptococcal species* also produce leukocidins. As a further example, strains of *B. fragilis* produce superoxide dismutase, which converts superoxide anions to hydrogen peroxide; strains of *E. coli* produce catalase, which reduces hydrogen peroxide to water, thereby rendering possible a synergism between these two organisms. A wide variety of other virulence factors have been identified.

The most important structural virulence factor is bacterial endotoxin. Endotoxin is derived from the lipopolysaccharide outer membrane that is found in virtually all Gram negative bacteria. Endotoxin induces an extensive array of biological effects. It is understood directly to stimulate the complement cascade, to provoke platelet aggregation, to induce fever, to activate phagocytosis and the immune system, and to stimulate the synthesis of numerous cytokines. Kremer, et al., "Interleukin-1, -6 and tumor necrosis factor-alpha release is down-regulated in whole blood from septic patients", Acta Haemmatol. 95(3-4):268-273, 1996.

Factors relevant to host susceptibility include the ease of entry by which a microorganism first gains access to the host, the impediments placed in the microorganism's path as it spreads within the host, and the ability of the host ultimately to contain the invasion before suffering substantial injury. Certain hosts are known to be more vulnerable than others. Newborns, for example, are particularly prone to severe infections and sepsis. Similarly, pediatric patients can develop sepsis in response to bacterial infections that are much more benign in the adult population. Infections in the elderly are also more likely to progress to sepsis than similar infections in younger patients. Certain pathological conditions are also understood to increase the host's susceptibility to infections and sepsis. Severe trauma, such as that which characterizes - major bums, predisposes the patient to microbial infections and sepsis to such an extent that these patients are considered immunocompromised hosts.

It would be desirable to identify those members of vulnerable populations at even more risk for overwhelming infection and its systemic consequences. For example, the newborn with a high temperature must be evaluated for foci of severe infection. This evaluation can include invasive measures such as lumbar puncture in order to rule out meningitis. Often the febrile newborn requires hospitalization and treatment with broad spectrum antibiotics until a source of the fever has been determined. If a subgroup of the newborn population could be identified as having greater risk or less risk of overwhelming infection, diagnostic and therapeutic measures could be tailored to the degree of risk. Lumbar puncture could be restricted to the high-risk infant, for example. Brik, et al., "Evaluation of febrile infants under 3 months of age: is routine lumbar puncture warranted?" Isr. J. Med. Sci. 33(2):93-97, 1997. Or, for example, low risk infants could be managed as outpatients or discharged quickly from the hospital, offering an important cost-saving in this era of managed care. Durongpisitkul, et al., "The appropriateness of early discharge of hospitalized children with suspected sepsis", J. Fam. Pract. 44(1):91-96, 1997. Infants or children at particular risk for certain severe systemic infections could be treated with infection-specific agents, or could be treated earlier or more aggressively.

Host defenses represent an important variable in determining the severity of a clinical infection. Non-specific host defenses serve to limit the initial extent of microbial invasion. Examples include the epiglottis mechanism of the trachea, the vibrissae of the nasal airway, the alveolar macrophage system and the acid environment of the stomach. More specific responses are set into motion on the cellular level once tissue injury or microbial contamination take place. As part of this specific response, the phagocytic-inflammatory components of host defense are initially mobilized with trauma or with the invasion of infecting agents. Phagocytosis and inflammation are intended to contain and destroy the organisms before they gain sufficient systemic access to cause a clinically significant infection. When a small scale infection is localized by these mechanisms, the clinical phenomena of cellulitis or abscess formation result. With more extensive microbial contamination, effective local containment may not be possible. Nonetheless, such containment is the goal of the phagocytic-inflammatory system of host defense.

A multitude of cellular functions contribute to the phase of specific host defense. First and foremost, in response to microbial invasion the host sets in motion the components of inflammation. Only when the stimulus of invading microorganisms becomes sufficiently pronounced do these inflammatory responses rise to the level of clinical infection. Clinical infection then becomes recognizable through the constellation of inflammatory responses that are responsive to the presence of the microorganisms. Rather than a specific response to a particular invader, clinical infection represents a set of nonspecific inflammatory responses elicited by every injury and every microbial contamination. In the ongoing presence of bacteria, the insult is active and progressive, providing a sustained injury that drives the inflammatory response until the offending agents are eradicated.

An early component of this inflammatory response is the complement cascade. This system is understood to be activated by various mechanisms of local tissue injury or microvascular trauma and disruption, leading to the release of opsonins and chemotactic signals that are complement cleavage products having the effect of attracting phagocytes and facilitating their functioning. Mast cells release inflammatory proteins such as kinins and histamines that increase vascular permeability and thus facilitate the access of intravascular proteins and cells into the affected area. Neutrophils are the first phagocytic cells to arrive on the scene. About 24 hours afterwards, activated macrophages arrive.

Macrophages are derived from monocytes that enter the tissues from the bloodstream. Monocytes recruited into the tissues differentiate into macrophages and become activated. In the activated state, macrophages produce a large number of inflammatory and cytokine proteins. An important cytokine released by the activated macrophage is TNF, which has autocrine and paracrine effects. TNF provides auto-stimulation to monocytes and macrophages to maintain full activation. TNF further stimulates neutrophils to full activation. In acute inflammation such as that found with acute infection, the activated neutrophil acts as the primary phagocyte, responsible for ingesting and killing the invading organisms. These cells may further release free oxygen radicals and lysosomal enzymes into the tissue fluid, causing extracellular killing of pathogens. Side-effects of the release of these cellular cytotoxic products include tissue necrosis, further inflammation and the activation of the coagulation cascade. Furthermore, neutrophils themselves are killed as these processes progress. The end result of this localized response to microbial invasion, with liquified necrotic cells and necrotic tissue, is known clinically as pus.

At the perimeter of the wound, surrounding the central core of necrotic material and cellular debris, additional biological processes are taking place intended to wall off or restrict the penetration of viable microorganisms into unaffected tissues. More neutrophils are attracted from adjacent microvessels by the release of complement cleavage products and TNF. Platelets and coagulation proteins are also activated in the adjacent microcirculation, leading to localized thrombosis. Platelets activated during the process of thrombosis produce thromboxane A2 by way of the cyclooxygenase-thromboxane synthetase pathway of prostaglandin biosynthesis. Thromboxane A2 is a potent vasoconstrictor. The combination of obstruction and vasoconstriction diminishes the inflow of circulation into the localized area of infection, but also blocks the access of pathogens to the general circulation. Activated neutrophils attracted to the periphery of the wound marginate within the microvasculature, leading to endothelial damage, increased vascular permeability and subsequent exudation of cells and serum proteins into the tissue space.

These serum components that leak into the tissues from the microvessels serve the additional function of bringing the building-blocks of wound healing into the infected area, first fibrin, albumin and globulin, and later fibroblasts. Circulating fibroblasts are attracted into the tissues by the growth factors secreted by the activated macrophages within the infected area. Fibroblasts, in turn, produce collagen, a protein that is the basis of scar tissue. If an infection becomes chronic, with the host unable completely to eliminate the pathogen, the infected area ultimately becomes surrounded by a wall of scar tissue formed by the processes of wound healing. In the context of acute or chronic infection, wound healing mechanisms help prevent the escape of the pathogen from the local area into the more general system.

Macrophages provide the connection between the local containment aspect of host defense and the systemic response. Activated macrophages release numerous secretion products, including cytokines that have systemic as well as local effects. Nathan, "Secretory products of macrophages," J. Clin. Invest. 79:319-326, 1987. The severity of the local inflammatory process may be extreme, due to magnitude of microbial inoculation or microbial virulence, so that the normal autocrine or paracrine mediators of inflammation come to have systemic effect. Systemic dissemination of pathogens or mediators of inflammation result in the host response termed sepsis.

Interleukin-1 (IL-1) is a cytokine released by the macrophage that can be disseminated systemically and induce a systemic response to local injury or infection. IL-1, when locally released, diffuses into the circulation, where it is ultimately carried to the hypothalamus. There, it acts to stimulate the production of prostaglandin-E which acts as an inflammatory mediator and an endogenous pyrogen. IL-1 is known to incite a variety of other systemic responses: it mobilizes neutrophils, stimulates liver production of acute phase proteins and complements, and interacts with tumor necrosis factor (TNF) to amplify the effects of TNF. Dinarello, "Interleukin-1," Rev. Infect. Disease 6:51-94, 1984. IL-1 further interacts with other cytokines and growth factors, for example mediating the sepsis induced changes in IGF and the accompanying changes in muscle protein synthesis. Lang, et al, "IL-1 receptor antagonist attentuates sepsis-induced alterations in the IGF system and protein synthesis", Am. J. Physiol. 270(3 Pt 1):E430-437, 1996; Lang, et al, "Role of central IL-1 in regulating peripheral IGF-I during endotoxemia and sepsis", Am. J. Physiol. 272(4 Pt 2):R956-962, 1998. IL-1 is also responsible for the increases in circulating eicosanoid levels, levels of IL-6 and levels of TNF. Slotman, et al, "Interleukin-1 mediates increased plasma levels of eicosanoids and cytokines in patients with sepsis syndrome", Shock 4(5):318-323, 1995; Slotman, et al, "Unopposed interleukin-1 is necessary for increased plasma cytokine and eicosanoid levels to develop in severe sepsis", Ann. Surg. 226(1):77-84, 1997.

The prior art further discloses a link between the Taql polymorphism in IL-1 beta and a tandem repeat polymorphism in exon 2 of IL-1RA and the susceptibility to sepsis. Fang, et al, "Genomic polymorphisms of the interleukin-1 gene family in patients suffering from severe sepsis" Anesthesiology (Hagerstown) 87(S3): A264, 1997. Fang, et al, "IL-1RA genomic polymorphism associated with susceptibility to and outcome of severe sepsis", Acta Anaesthesiologica Scandinavica 40(S109):236, 1996.

EP 0 769 560, WO 98/15653, and WO 98/40517 disclose primers and probes suitable for detecting polymorphisms of the interleukin-1 gene family. However, they do not disclose a link of these polymorphisms with severe sepsis.

When the systemic effects of host defense response accompany a microbial invasion, the condition is termed "sepsis." Standard definitions do not exist for such terms as sepsis, septicemia, septic syndrome and septic response. Most connotations of these terms associate them with severe systemic infection. Traditionally, the most common offending agents were thought to be gram negative bacteria; more recently it has been observed that patients can have characteristic responses of sepsis without a clearly identifiable inciting microbe. The term sepsis has thus come to be associated with any systemic response to overwhelming infection or other severe insult. Kelly, et al, "Is circulating endotoxin the trigger for the systemic inflammatory response syndrome seen after injury?" Ann. Surg. 225(5):530-541; discussion 542-543, 1997.

The term "systemic inflammatory response syndrome" (SIRS) has been applied to a set of responses consistent with what is commonly understood to be sepsis. American College of Chest Physicians/Society of Critical Care Medicine Consensus Conference, "Definition for sepsis and organ failure and guidelines for the use of innovative therapies in sepsis," Crit. Care Med. 20:864, 1992. The elements of this syndrome revolve around certain clinical findings, including temperature, heart rate, respiratory rate or PaC02 and white cell count. SIRS criteria have been applied in prospective studies considering the prognosis of patients with septic-related diagnoses.

The SIRS criteria are thought by some authors to be too broad to have clinical value. One study followed 1101 patients admitted to intensive care units, finding about half of the admissions to have manifestations of SIRS, with 16% to have findings consistent with established sepsis, 5% to have findings consistent with severe sepsis and 6% to be in the state of septic shock. Salvo, et al., "The Italian SEPSIS study: preliminary results on the incidence and evolution of SIRS, sepsis, severe sepsis, and septic shock", *Intensive Care Med*. 21(Supple 2):S244-249, 1995. The mortality rates rise with the severity of the septic elements: about one-fourth of those patients with SIRS died, 36% with sepsis, 52% with severe sepsis, and 81 % with septic shock. Late mortality after sepsis and septic shock is equally poor, with only 30% surviving the first year after hospital admission. Schoenberg, et al. "Outcome of patients with sepsis and shock after ICU treatment", Langenbecks Arch. Surg. 383(1):44-48, 1998.

The Salvo study introduced a set of gradations in inflammatory severity that parallels the staging system proposed by Siegel et al. in the earlier surgical literature. Siegel et al., "Physiologic and metabolic correlations in human sepsis," Surg. 86:163-193, 1979. According to Siegel's system, the extent of the septic response is assessed according to four criteria: hyperdynamic cardiac parameters, reduced peripheral vascular resistance, narrowed arteriovenous oxygen difference, and abnormal serum lactic acid levels. Within this staging system, Stage A is characterized by a physiologic stress response, Stage B represents an exaggerated stress response, Stage C is the onset of septic shock and Stage D is low output failure and established shock. Individual patients do not necessarily progress sequentially from one stage to another. A patient can decompensate from Stage A to Stage C with no Stage B interval. Alternatively, with modem technology, a patient can be sustained in Stage B for a prolonged time, setting the stage for a number of sepsis-related sequelae such as multiple organ failure even though no frank shock has supervened. It would be desirable to identify those patients whose septic course is more likely to be progressive. This would allow early and aggressive therapies to be directed towards those patients who face the most dire prognoses. Horn, K.D. "Evolving strategies in the treatment of sepsis and systemic inflammatory response syndrome (SIRS)", QJM 91(4):265-277, 1998. Similarly, if a patient is likely to remain in a prolonged Stage B, supportive measures - can be instituted at early stages to forestall the consequences of multiple organ failure.

Alternate hypotheses have been proposed to explain the progression of sepsis and the occurrence of sepsis-related sequelae that can be as lethal as Stage D septic shock. One hypothesis suggests that the primary defect in sepsis is mitochondrial injury, whereby the mitochondria are unable to metabolize oxygen and related substrates. Mela et al., "Defective oxidative metabolism of rat liver mitochondria in hemorrhagic and endotoxin shock," Am. J. Physiol. 220:571-580, 1971. A second hypothesis focuses on the parallels between the systemic septic response and the local response to tissue infection and injury. This hypothesis is supported by an extensive body of experimental and clinical literature. According to this view, systemic complement activation and systemic macrophage activation lead to systemic neutrophil activation, in analogy to the interrelated local behaviors of complement, macrophages and neutrophils. Schirmer et al., "Complement activation produces hemodynamic changes characteristic of sepsis," Arch. Surg. 123:316-321, 1989; Schirmer et al., "Recombinant human TNF produces hemodynamic changes characteristic of sepsis and endotoxemia," Arch. Surg. 124:445-448, 1989.

When neutrophils are systemically activated, their actions are diffuse and unchanneled. Systemic neutrophil activation also entails diffuse neutrophil margination. In this situation, the neutrophils attach to the endothelium of vessels throughout the body and exert their effects on all tissues they encounter. Endothelial injury results from secretion of neutrophil products, leading to increased vascular permeability. As neutrophils attach to the endothelium and enter the tissues, they also release oxygen free radicals and lysozymal enzymes which contribute to a systemic inflammatory response. Release of these products into the bloodstream catalyzes further systemic responses. Entry of neutrophils into local tissues previously unaffected by infection allows disseminated tissue damage to take place.

Endothelial injury from the secreted products of activated neutrophils further results in platelet activation and induction of the coagulation cascade. Sutton, et al, "Endothelial structural integrity is maintained during endotoxic shock in an interleukin-1 type 1 receptor knockout mouse" Shock 7(2):105-110, 1997 Thromboxane A2 is thereupon released. As a result of these processes, plugs are formed in the microvascular system from the combination of neutrophils, platelets and fibrin. These plugs, combined with the vasoconstrictive effects of thromboxane, cause focal tissue ischemia. Chang, et al., "Interleukin-1 in ischemia-reperfusion acute lung injury", Am. J. Respir. Crit. Care Med. 156(4 Pt 1):1230-1234, 1997. Focal ischemia in tissues leads to focal necrosis. Hinshaw, L.B. "Sepsis/septic shock: participation of the microcirculation: an abbreviated review", Crit. Care Med. 24(6):1072-1078, 1996. A physiological paradox comes to exist, where microcirculatory ischemia exists despite the presence of a hyperdynamic circulation.

Tissue necrosis, both locally and distantly, in its turn provides a stimulus for further inflammation. Rapid evolution of these processes can lead to the progression from Stage C sepsis to Stage D sepsis, with a fatal outcome common. Alternatively, if Stage B sepsis is prolonged in the face of these microcirculatory events, focal but disseminated tissue necrosis can extend to culminate in multiple organ failure.

It is understood that the systemic inflammatory response has beneficial effects as part of the host's immune system. Ertel, et al., "Downregulation of proinflammatory cytokine release in whole blood from septic patients", Blood 85(5):1341-1347, 1995. Cytokine release has been identified following surgical procedures, with more severe operative trauma occasioning more extensive release Pruitt et al., "Interleukin-1 and interleuin-1 antagonist [IL-1RN] in sepsis, systemic inflammatory response syndrome and septic shock," Shock 3:235-251, 1995. Conversely, defective response in cytokine production can lead to inadequate immune response to stress or infectious insult. Samson, et al., "Elevated interleukin-1 receptor antagonist levels in pediatric sepsis syndrome" J. Pediatr. 131(4):587-591, 1997. Neonates with sepsis, for example, have been found to have lower levels of serum IL-1 and higher levels of IL-1RN vs. normal controls. Atici, et al., "Serum interleukin-1 beta in neonatal sepsis", Acta Pediatr. 85(3):371-374, 1996; de Bont, et al., "Increased plasma concentrations of interleukin-1 receptor antagonist in neonatal sepsis", Pediatr. Res. 37(5):626-629, 1995.

It is therefore desirable to identify those whose interleukin immune feedback systems make them more vulnerable to overwhelming, initially occult sepsis. However, it is further recognized that an excessively vigorous systemic inflammatory response comprises the patterns of sepsis that culminate in such disastrous events as disseminated intravascular coagulation, multiple organ failure and cardiovascular collapse. Aikawa has termed the excessive production of cytokines that culminates in this generalized autoinflammatory reaction "cytokine storm." Aikawa, N. "Cytokine storm in the pathogenesis of multiple organ dysfunction syndrome associated with surgical insults" Nippon Geka Gakkai Zasshi 97(9):771-777, 1996. It would be clinically useful to identify patients at heightened risk for exaggerated inflammatory response who may therefore be prone to its undesirable sequelae.

Current understanding has highlighted the role played in systemic inflammation by various cytokines. Blackwell and Christman, "Sepsis and cytokines: current status", Br. J. Anaesth. 77(1):110-117, 1996. Excessive IL-1 production, for example, has been linked to the development of hypotension, shock, adult respiratory distress syndrome (ARDS), multiple organ failure, hematological abnormalities and death in patients and experimental animals with sepsis. Pruitt et al., *supra*. IL-1 and TNF have been implicated in producing the metabolic alterations found in sepsis and injury. Ling, et al., "Differential effects on interleukin-1 receptor antagonist in cytokine- and endotoxin-treated rats", Am. J. Physiol. 268(2 Pt 1):E255-261, 1995. Similarly, trauma patients have been found to demonstrate elevated levels of inflammatory mediators, consistent with the clinical features of inflammation in these conditions. Endo, et al. "Plasma levels of interleukin-1 receptor antagonist (IL-1ra) and severity of illness in patients with burns), J. Med. 27(1-2):57-71, 1996. Cytokines, particularly IL-1 and TNF, are identified as coordinating the cascade of interactions between leukocytes and endothelial cells which result in the types of tissue damage discussed above as characteristic of sepsis. Shanley, et al., "The role of cyotkines and adhesion molecules in the development of inflammatory injury", Mol. Med. Today 1(1):40-45, 1995. The presence of thrombin is understood to stimulate further production of IL-1 and TNF, thereby perpetuating the cycles of thrombosis and DIC that can accompany sepsis. Hoffman and Cooper, "Thrombin enhances monocyte secretion of tumor necrosis factor and interleukin-1 beta by two distinct mechanisms", Blood Cells Mol. Dis. 21(2):156-167, 1995; Gando, et al., "Cytokines, soluble thrombomodulin and disseminated intravascular coagulation in patients with systemic inflammatory response syndrome" Thromb. Res. 80(6):519-526, 1995.

Many current approaches for treating sepsis and its sequelae attempt to modulate cytokine interactions within the inflammatory cascade. Since IL-1 and TNF have been identified as circulating factors that integrate and perpetuate these effects, therapies designed to antagonize the effects of these agents can be designed to have clinical utility in ameliorating the sequences involved in sepsis. For example, IL-1 has been identified as playing an important role in Group B streptococcal sepsis and septic shock in the newborn; it is suggested that IL1-RN treatment may ameliorate the cardiovascular alterations associated with this disease in the newborn population. - Vallette, et al., "Effect of an interleukin-1 receptor antagonist on the hemodynamic manifestations of group B streptococcal sepsis", Pediatr. Res. 38(5):704-708, 1995. Other data suggest, however, that specific cytokine inhibitors may not be effective in modulating inflammation induced by gram-negative bacterial products. Paris, et al., "Effect of interleukin-1 receptor antagonist and soluble tumor necrosis factor receptor in animal models of infection", J. Infect. Dis. 171(1):161-169, 1995. Therefore it would be useful to identify those diseases where cytokine modification is likely to work, and those where it is likely to be ineffective or hazardous.

It would furthermore be clinically advantageous to identify those patients with sepsis in whom early intervention strategies may forestall potentially devastating complications. For example, elevated levels of IL-1 have been identified as markers for poor prognosis in patients with ARDS, a common concomitant of sepsis. Meduri, et al., "Persistent elevation of inflammatory cytokines predicts a poor outcome in ARDS. Plasma IL-1 beta and IL-6 levels are consistent and efficient predictors of outcome over time", Chest 107(4):1062-1073, 1995. Determining whether a patient falls into the subgroup destined for a poor outcome can motivate the clinician to undertake early and perhaps more ambitious therapies for the ARDS, for example, early glucacorticoid treatment or early institution of extracorporeal membrane oxygenator. Headley, et al, "Infections and the inflammatory response in acute respiratory distress syndrome", Chest 111(5):1306-1321, 1997. Bonten, et al., "The systemic inflammatory response in the development of ventilator-associated pneumonia", Am. J. Respir. Crit. Care Med. 156(4 Pt 1):1105-1113, 1997. Similarly, a patient at high risk for poor outcome may merit early, aggressive, continuous and/or multiple antibiotic treatment. Mercer-Jones, et al., "Continuous antibiotic treatment for experimental abdominal sepsis: effects on organ inflammatory cytokine expression and neutrophil sequestration" Br. J. Surg. 85(3):385-389, 1998. Steroids may be indicated to treat the global inflammatory response in those patients who are identified as inflammation overreactors. Jones and Lowes, "The systemic inflammatory response syndrome as a predictor of bacteraemia and outcome from sepsis", QJM 89(7):515-522, 1996. Lefering and Neugebauer, "Steroid controversy in sepsis and septic shock: a meta-analysis", Crit. Care Med. 23(7):1294-1303, 1995. Plasmapherisis may be appropriate to remove the inflammatory elements from the septic patient's bloodstream in those who are prone to exaggerated inflammatory response. Haupt, et al., "Selective cytokine release induced by serum and separated plasma from septic patients", Eur. J. Surg. 162(10):769-776, 1996; Stegmayr, B.G. "Plasmapheresis in severe sepsis or septic shock", Blood Purif. 14(1):94-101, 1996. Manipulating the complement system may provide an additional strategy for treating the patient with severe inflammatory response in sepsis. Kirschfink, M. "Controlling the complement system in inflammation", Immunopharmacology 38(1-2):51-62, 1997. Or, realizing the additional inflammatory burden imposed by surgery in certain septic patients may further provide the clinician information about the timing of surgical interventions in sepsis, and will guide the clinician in possible forms of adjuvant therapy.

Recognizing these potential prognostic and therapeutic implications of cytokine release has led investigators and clinicians to measure cytokine levels and try to correlate them with clinical situations. van der Poll, et al, "Anti-inflammatory cytokine responses during clinical sepsis and experimental endotoxemia: sequential measurements of plasma soluble interleukin (IL)-1 receptor type II, IL-10, and IL-13", J. Infect. Dis. 175(1):118-122, 1997. For example, IL-1ra has been measured in high risk neonates and noted to be elevated one or more days before the onset of clinical sepsis (Kuester, H. et al., (1998) The Lancet 352:1271-1277). Unfortunately, under varying clinical circumstances, there has been marked variability in the data. For example, during the development of organ failure and death as a result of intra-abdominal sepsis, levels of proinflammatory mediators and their endogenous antagonists vary considerably. Wakefield, et al., "Proinflammatory mediator activity, endogenous antagonists and the systemic inflammatory response in intra-abdominal sepsis. Scottish Sepsis Intervention Group", Br. J. Surg. 85(6):818-825, 1998. Some authors find that IL-1 levels correlate positively with poor prognosis in sepsis, (Thijs and Hack, "Time course of cytokine levels in sepsis", Intensive Care Med. 21 (Suppl. 2):S258-263, 1995), while others fail to find this correlation. Goldie, et al., "Natural cytokine antagonists and endogenous antiendotoxin core antibodies in sepsis syndrome", JAMA 274(2):172-177, 1995.

### Genetics of the IL-1 Gene Cluster

The IL-1 gene cluster is on the long arm of chromosome 2 (2q13) and contains at least the genes for IL-1α (IL-1A), IL-1β (IL-1B), and the IL-1 receptor antagonist (IL-1RN), within a region of 430 Kb (Nicklin, et al. (1994) Genomics, 19: 382-4). The agonist molecules, IL-1α and IL-1β, have potent pro-inflammatory activity and are at the head of many inflammatory cascades. Their actions, often via the induction of other cytokines such as IL-6 and IL-8, lead to activation and recruitment of leukocytes into damaged tissue, local production of vasoactive agents, fever response in the brain and hepatic acute phase response. All three IL-1 molecules bind to type I and to type II IL-1 receptors, but only the type I receptor transduces a signal to the interior of the cell. In contrast, the type II receptor is shed from the cell membrane and acts as a decoy receptor. The receptor antagonist and the type II receptor, therefore, are both anti-inflammatory in their actions.

Inappropriate production of IL-1 plays a central role in the pathology of many autoimmune and inflammatory diseases, including rheumatoid arthritis, inflammatory bowel disorder, psoriasis, and the like. In addition, there are stable inter-individual differences in the rates of production of IL-1, and some of this variation may be accounted for by genetic differences at IL-1 gene loci. Thus, the IL-1 genes are reasonable candidates for determining part of the genetic susceptibility to inflammatory diseases, most of which have a multifactorial etiology with a polygenic component.

Certain alleles from the IL-1 gene cluster are known to be associated with particular disease states. For example, IL-1RN (VNTR) allele 2 has been shown to be associated with osteoporosis (U.S. Patent No. 5,698,399), nephropathy in diabetes mellitus (Blakemore, et al. (1996) Hum. Genet. 97(3): 369-74), alopecia areata (Cork, et al., (1995) J. Invest. Dermatol. 104(5 Supp.): 15S-16S; Cork et al. (1996) Dermatol Clin 14: 671-8), Graves disease (Blakemore, et al. (1995) J. Clin. Endocrinol. 80(1): 111-5), systemic lupus erythematosus (Blakemore, et al. (1994) Arthritis Rheum. 37: 1380-85), lichen sclerosis (Clay, et al. (1994) Hum. Genet. 94: 407-10), and ulcerative colitis (Mansfield, et al. (1994) Gastoenterol. 106(3): 637-42)).

In addition, the IL-1A allele 2 from marker -889 and IL-1B (TaqI) allele 2 from marker +3954 have been found to be associated with periodontal disease (U.S. Patent No. 5,686,246; Kornman and diGiovine (1998) Ann Periodont 3: 327-38; Hart and Kornman (1997) Periodontol 2000 14: 202-15; Newman (1997) Compend Contin Educ Dent 18: 881-4; Kornman et al. (1997) J. Clin Periodontol 24: 72-77). The IL-1A allele 2 from marker -889 has also been found to be associated with juvenile chronic arthritis, particularly chronic iridocyclitis (McDowell, et al. (1995) Arthritis Rheum. 38: 221-28). The IL-1B (TaqI) allele 2 from marker +3954 of IL-1B has also been found to be associated with psoriasis and insulin dependent diabetes in DR3/4 patients (di Giovine, et al. (1995) Cytokine 7: 606; Pociot, et al. (1992) Eur J. Clin. Invest. 22: 396-402). Additionally, the IL-1RN (VNTR) allele 1 has been found to be associated with diabetic retinopathy (see USSN 09/037472, and PCT/GB97/02790). Furthermore allele 2 of IL-1RN (VNTR) has been found to be associated with ulcerative colitis in Caucasian populations from North America and Europe (Mansfield, J. et al., (1994) Gastroenterology 106: 637-42). Interestingly, this association is particularly strong within populations of ethnically related Ashkenazi Jews (PCT WO97/25445).

### Genotype Screening

Traditional methods for the screening of heritable diseases have depended on either the identification of abnormal gene products (e.g., sickle cell anemia) or an abnormal phenotype (e.g., mental retardation). These methods are of limited utility for heritable diseases with late onset and no easily identifiable phenotypes such as, for example, vascular disease. With the development of simple and inexpensive genetic screening methodology, it is now possible to identify polymorphisms that indicate a propensity to develop disease, even when the disease is of polygenic origin. The number of diseases that can be screened by molecular biological methods continues to grow with increased understanding of the genetic basis of multifactorial disorders.

Genetic screening (also called genotyping or molecular screening), can be broadly defined as testing to determine if a patient has mutations (or alleles or polymorphisms) that either cause a disease state or are "linked" to the mutation causing a disease state. Linkage refers to the phenomenon wherein DNA sequences which are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population. Indeed, where multiple polymorphisms in a given chromosomal region are found to be in linkage disequilibrium with one another, they define a quasi-stable genetic "haplotype." In contrast, recombination events occurring between two polymorphic loci cause them to become separated onto distinct homologous chromosomes. If meiotic recombination between two physically linked polymorphisms occurs frequently enough, the two polymorphisms will appear to segregate independently and are said to be in linkage equilibrium.

While the frequency of meiotic recombination between two markers is generally proportional to the physical distance between them on the chromosome, the occurrence of "hot spots" as well as regions of repressed chromosomal recombination can result in discrepancies between the physical and recombinational distance between two markers. Thus, in certain chromosomal regions, multiple polymorphic loci spanning a broad chromosomal domain may be in linkage disequilibrium with one another, and thereby define a broad-spanning genetic haplotype. Furthermore, where a disease-causing mutation is found within or in linkage with this haplotype, one or more polymorphic alleles of the haplotype can be used as a diagnostic or prognostic indicator of the likelihood of developing the disease. This association between otherwise benign polymorphisms and a disease-causing polymorphism occurs if the disease mutation arose in the recent past, so that sufficient time has not elapsed for equilibrium to be achieved through recombination events. Therefore identification of a human haplotype which spans or is linked to a disease-causing mutational change, serves as a predictive measure of an individual's likelihood of having inherited that disease-causing mutation. Importantly, such prognostic or diagnostic procedures can be utilized without necessitating the identification and isolation of the actual disease-causing lesion. This is significant because the precise determination of the molecular defect involved in a disease process can be difficult and laborious, especially in the case of multifactorial diseases such as inflammatory disorders.

Indeed, the statistical correlation between an inflammatory disorder and an IL-1 polymorphism does not necessarily indicate that the polymorphism directly causes the disorder. Rather the correlated polymorphism may be a benign allelic variant which is linked to (i.e. in linkage disequilibrium with) a disorder-causing mutation which has occurred in the recent human evolutionary past, so that sufficient time has not elapsed for equilibrium to be achieved through recombination events in the intervening chromosomal segment. Thus, for the purposes of diagnostic and prognostic assays for a particular disease, detection of a polymorphic allele associated with that disease can be utilized without consideration of whether the polymorphism is directly involved in the etiology of the disease. Furthermore, where a given benign polymorphic locus is in linkage disequilibrium with an apparent disease-causing polymorphic locus, still other polymorphic loci which are in linkage disequilibrium with the benign polymorphic locus are also likely to be in linkage disequilibrium with the disease-causing polymorphic locus. Thus these other polymorphic loci will also be prognostic or diagnostic of the likelihood of having inherited the disease-causing polymorphic locus. Indeed, a broad-spanning human haplotype (describing the typical pattern of co-inheritance of alleles of a set of linked polymorphic markers) can be targeted for diagnostic purposes once an association has been drawn between a particular disease or condition and a corresponding human haplotype. Thus, the determination of an individual's likelihood for developing a particular disease of condition can be made by characterizing one or more disease-associated polymorphic alleles (or even one or more disease-associated haplotypes) without necessarily determining or characterizing the causative genetic variation.

A means for measuring a patient's propensity for exaggerated inflammatory response as an indicator of his or her response to septic stimuli is needed.

### 2. SUMMARY OF THE INVENTION

In one aspect, the invention features assays for determining a subject's susceptibility to developing sepsis or prognosticating on the rapidity and/or ultimate progression of sepsis in that subject. In one embodiment, the method comprises the step of genotyping a nucleic acid sample obtained from the subject to determine whether the subject's DNA contains at least one allele of an IL-1 genetic pattern that leads to a dysregulated inflammatory response. In a preferred embodiment, the IL-1 genetic pattern that leads to a dysregulated inflammatory response is an IL-1 pattern 2. In another preferred embodiment, the allele of an IL-1 genetic pattern is detected by one of the following methods: 1) performing a hybridization reaction between the nucleic acid sample and a probe or probes that are capable of hybridizing to a particular allele in the subject; 2) sequencing at least a portion of at least one allele; or 3) determining the electrophoretic mobility of at least one allele. In another preferred embodiment, an allele of an IL-1 genetic pattern that leads to a dysregulated inflammatory response is subject to an amplification step, prior to performance of the detection step. Preferred amplification steps are selected from the group consisiting of: the polymerase chain reaction (PCR), the ligase chain reaction (LCR), strand displacement amplification (SDA), cloning, and variations of the above (e.g. RT-PCR and allele specific amplification). In a particularly preferred embodiment, the sample is hybridized with a set of primers, which hybridize 5' and 3' to a sense or antisense sequence of an allele of an IL-1 genetic pattern that leads to a dysregulated inflammatory response and is subject to a PCR amplification.

In another aspect, the invention features kits for performing the above-described assays. The kit can include DNA sample collection means and a means for determining at least one allele of an IL-1 genetic pattern that leads to a dysregulated inflammatory response of the subject. The kit may also comprise control samples or standards.

Information obtained using the assays and kits described herein (alone or in conjunction with information on another genetic defect or environmental factor, which contributes to sepsis) is useful for predicting whether a subject is likely to develop sepsis. In addition, the information alone or in conjunction with information on another genetic defect contributing to sepsis (the genetic profile of sepsis) allows customization of sepsis therapy to the individual's genetic profile. For example, this information can enable a doctor to: 1) more effectively prescribe a drug that will address the molecular basis of the cascade resulting in sepsis; and 2) better determine the appropriate dosage of a particular sepsis drug for a particular patient.

The ability to target patient populations expected to show the highest clinical benefit, can enable: 1) the repositioning of marketed drugs with disappointing market results; 2) the rescue of drug candidates whose clinical development has been discontinued as a result of safety or efficacy limitations, which are patient subgroup-specific; and 3) an accelerated and less costly development for drug candidates and more optimal drug labeling.

In a further aspect, the invention features methods for treating or preventing the development of sepsis in a subject by administering to the subject an appropriate therapeutic of the invention. In still another aspect, the invention provides *in vitro* or *in vivo* assays for screening test compounds to identify sepsis therapeutics.

Other features and advantages of the invention will be apparent from the following detailed description and claims.

### 3. DETAILED DESCRIPTION OF THE INVENTION

### 3.1 Definitions

For convenience, the meaning of certain terms and phrases employed in the specification, examples, and appended claims are provided below.

The term "aberrant activity" as applied to an activity of a polypeptide such as IL-1, refers to an activity which differs from the activity of a native polypeptide or which differs from the activity of the polypeptide in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in an activity. For example an aberrant polypeptide can interact with a different target peptide. A cell can have an aberrant IL-1 activity due to overexpression or underexpression of an IL-1 locus gene encoding an IL-1 locus polypeptide.

The term "allele" refers to the different sequence variants found at different polymorphic regions. For example, IL-1RN (VNTR) has at least five different alleles. The sequence variants may be single or multiple base changes, including without limitation insertions, deletions, or substitutions, or may be a variable number of sequence repeats.

The term "allelic pattern" refers to the identity of an allele or alleles at one or more polymorphic regions. For example, an allelic pattern may consist of a single allele at a polymorphic site, as for IL-1RN (VNTR) allele 1, which is an allelic pattern having at least one copy of IL-1RN allele 1 at the VNTR of the IL-1RN gene loci. Alternatively, an allelic pattern may consist of either a homozygous or heterozygous state at a single polymorphic site. For example, IL1-RN (VNTR) allele 2,2 is an allelic pattern in which there are two copies of the second allele at the VNTR marker of IL-1RN and that corresponds to the homozygous IL-RN (VNTR) allele 2 state. Alternatively, an allelic pattern may consist of the identity of alleles at more than one polymorphic site.

The term "antibody " as used herein is intended to refer to a binding agent including a whole antibody or a binding fragment thereof which is specifically reactive with an IL-1B polypeptide. Antibodies can be fragmented using conventional techniques and the fragments screened for utility in the same manner as described above for whole antibodies. For example, F(ab)₂ fragments can be generated by treating an antibody with pepsin. The resulting F(ab)₂ fragment can be treated to reduce disulfide bridges to produce Fab fragments. The antibody of the present invention is further intended to include bispecific, single-chain, and chimeric and humanized molecules having affinity for an IL-1B polypeptide conferred by at least one CDR region of the antibody.

"Biological activity" or "bioactivity" or "activity" or "biological function", which are used interchangeably, for the purposes herein when applied to IL-1 means an effector or antigenic function that is directly or indirectly performed by an IL-1 polypeptide (whether in its native or denatured conformation), or by any subsequence (fragment) thereof. A biological activity can include binding, effecting signal transduction from a receptor, modulation of gene expression or an antigenic effector function.

As used herein the term "bioactive fragment of an IL-1 polypeptide" refers to a fragment of a full-length IL-1 polypeptide, wherein the fragment specifically mimics or antagonizes the activity of a wild-type IL-1 polypeptide. The bioactive fragment preferably is a fragment capable of interacting with an interleukin receptor.

"Cells", "host cells" or "recombinant host cells" are terms used interchangeably herein to refer not only to the particular subject cell, but to the progeny or potential progeny of such a cell. Because certain modifications may occur in succeeding generations due to either mutation or environmental influences, such progeny may not, in fact be identical to the parent cell, but is still included within the scope of the term as used herein.

A "chimera," "mosaic," "chimeric mammal" and the like, refers to a transgenic mammal with a knock-out or knock-in construct in at least some of its genome-containing cells.

The terms "control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the IL-1 gene cluster. However, where the sample to be tested is genomic DNA, the control sample is preferably a highly purified sample of genomic DNA.

The phrases "disruption of the gene" and "targeted disruption" or any similar phrase refers to the site specific interruption of a native DNA sequence so as to prevent expression of that gene in the cell as compared to the wild-type copy of the gene. The interruption may be caused by deletions, insertions or modifications to the gene, or any combination thereof.

The term "haplotype" as used herein is intended to refer to a set of alleles that are inherited together as a group (are in linkage disequilibrium) at statistically significant levels (p_{corr} < 0.05). As used herein, the phrase "an IL-1 haplotype" refers to a haplotype in the IL-1 loci.

An "IL-1 agonist" as used herein refers to an agent that mimics, upregulates (potentiates or supplements) or otherwise increases an IL-1 bioactivity or a bioactivity of a gene in an IL-1 biological pathway. IL-1 agonists may act on any of a variety of different levels, including regulation of IL-1 gene expression at the promoter region, regulation of mRNA splicing mechanisms, stabilization of mRNA, phosphorylation of proteins for translation, conversion of proIL-1 to mature IL-1 and secretion of IL-1. Agonists that increase IL-1 synthesis include: lipopolysaccharides, IL-1B, cAMP inducing agents, NfκB activating agents, AP-1 activating agents, TNF-α, oxidized LDL, advanced glycosylation end products (AGE), sheer stress, hypoxia, hyperoxia, ischemia reperfusion injury, histamine, prostaglandin E 2 (PGE2), IL-2, IL-3, IL-12, granulocyte macrophage-colony stimulating factor (GM-CSF), monocyte colony stimulating factor (M-CSF), stem cell factor, platelet derived growth factor (PDGF), complement C5A, complement C5b9, fibrin degradation products, plasmin, thrombin, 9-hydroxyoctadecaenoic acid, 13-hydroxyoctadecaenoic acid, platelet activating factor (PAF), factor H, retinoic acid, uric acid, calcium pyrophosphate, polynucleosides, c-reactive protein, α-antitrypsin, tobacco antigen, collagen, β-1 integrins, LFA-3, anti-HLA-DR, anti-IgM, anti- CD3, phytohemagglutinin (CD2), sCD23, ultraviolet B radiation, gamma radiation, substance P,. isoproterenol, methamphetamine and melatonin. Agonists that stabilize IL-1 mRNA include bacterial endotoxin and IL-1. Other agonists, that function by increasing the number of IL-1 type 1 receptors available, include IL-1, PKC activators, dexamethasone, IL-2, IL-4 and PGE2. Other preferred antagonists interfere or inhibit signal transduction factors activated by IL-1 or utilized in an IL-1 signal transduction pathway (e.g NFkB and AP-1, PI3 kinase, phospholipase A2, protein kinase C, JNK-1, 5-lipoxygenase, cyclooxygenase 2, tyrosine phosphorylation, iNOS pathway, Rac, Ras, TRAF). Still other agonists increase the bioactivity of genes whose expression is induced by IL-1, including: IL-1, IL-1Ra, TNF, IL-2, IL-3, IL-6, - IL-12, GM-CSF, G-CSF, TGF-β, fibrinogen, urokinase plasminogen inhibitor, Type 1 and type 2 plasminogen activator inhibitor, p-selectin (CD62), fibrinogen receptor, CD-11/CD18, protease nexin-1, CD44, Matrix metalloproteinase-1 (MMP-1),MMP-3, Elastase, Collagenases, Tissue inhibitor ofmetalloproteinases-1 (TIMP-1),Collagen, Triglyceride increasing Apo CIII, Apolipoprotein, ICAM-1, ELAM-1, VCAM-1, L-selectin, Decorin, stem cell factor, Leukemia inhibiting factor, IFNa,b,g, L-8, IL-2 receptor, IL-3 receptor, IL-5 receptor, *c-kit* receptor, GM-CSF receptor, Cyclooxygenase-2 (COX-2), Type 2 phospholipase A2, Inducible nitric oxide synthase (iNOS), Endothelin-1,3, Gamma glutamyl transferase, Mn superoxide dismutase, C-reactive protein, Fibrinogen, Serum amyloid A, Metallothioneins, Ceruloplasmin, Lysozyme, Xanthine dehydrogenase, Xanthine oxidase, Platelet derived growth factor A chain (PDGF), Melanoma growth stimulatory activity (*gro-a,b,g*), Insulin-like growth factor-1 (IGF-1), Activin A, Pro-opiomelanocortiotropin, corticotropin releasing factor, B amyloid precursor, Basement membrane protein-40, Laminin B1 and B2, Constitutive heat shock protein p70, P42 mitogen, activating protein kinase, ornithine decarboxylase, heme oxygenase and G-protein a subunit).

An "IL-1 antagonist" as used herein refers to an agent that downregulates or otherwise decreases an IL-1 bioactivity. IL-1 antagonists may act on any of a variety of different levels, including regulation of IL-1 gene expression at the promoter region, regulation of mRNA splicing mechanisms, stabilization of mRNA, phosphorylation of proteins for translation, conversion of proIL-1 to mature IL-1 and secretion of IL-1. Antagonists of IL-1 production include: corticosteroids, lipoxygenase inhibitors, cyclooxygenase inhibitors, γ-interferon, IL-4, IL-10, IL-13, transforming growth factor β (TGF-β), ACE inhibitors, n-3 polyunsaturated fatty acids, antioxidants and lipid reducing agents. Antagonists that destabilize IL-1mRNA include agents that promote deadenylation. Antagonists that inhibit or prevent phosphorylation of IL-1 proteins for translation include pyridinyl-imadazole compounds, such as tebufelone and compounds that inhibit microtubule formation (e.g. colchicine, vinblastine and vincristine). Antagonists that inhibit or prevent the conversion of proIL-1 to mature IL-1 include interleukin converting enzyme (ICE) inhibitors, such as εICE isoforms, ICE α, β, and γ isoform antibodies, CXrm-A, transcript X, endogenous tetrapeptide competitive substrate inhibitor, trypsin, elastase, chymotrypsin, chymase, and other nonspecific proteases. Antagonists that prevent or inhibit the scretion of IL-1 include agents that block anion transport. Antagonists that interefere with IL-1 receptor interactions, include: agents that inhibit glycosylation of the type I IL-1 receptor, antisense - oligonucleotides against IL-1RI, antibodies to IL-1RI and antisense oligonucleotides against IL-1RacP. Other antagonists, that function by decreasing the number of IL-1 type 1 receptors available, include TGF-β, COX inhibitors, factors that increase IL-1 type II receptors, dexamethasone, PGE2, IL-1 and IL-4. Other preferred antagonists interfere or inhibit signal transduction factors activated by IL-1 or utilized in an IL-1 signal transduction pathway (e.g NFkB and AP-1, PI3 kinase, phospholipase A2, protein kinase C, JNK-1, 5-lipoxygenase, cyclooxygenase 2, tyrosine phosphorylation, iNOS pathway, Rac, Ras, TRAF). Still other antagonists interfere with the bioactivity of genes whose expression is induced by IL-1, including: IL-1, IL-1Ra, TNF, IL-2, IL-3, IL-6, IL-12, GM-CSF, G-CSF, TGF-β, fibrinogen, urokinase plasminogen inhibitor, Type 1 and type 2 plasminogen activator inhibitor, p-selectin (CD62), fibrinogen receptor, CD-11/CD18, protease nexin-1, CD44, Matrix metalloproteinase-1 (MMP-1),MMP-3, Elastase, Collagenases, Tissue inhibitor of metalloproteinases-1 (TIMP-1),Collagen, Triglyceride increasing Apo CIII, Apolipoprotein, ICAM-1, ELAM-1, VCAM-1, L-selectin, Decorin, stem cell factor, Leukemia inhibiting factor, IFNa,b,g, L-8, IL-2 receptor, IL-3 receptor, IL-5 receptor, c-kit receptor, GM-CSF receptor, Cyclooxygenase-2 (COX-2), Type 2 phospholipase A2, Inducible nitric oxide synthase (iNOS), Endothelin-1,3, Gamma glutamyl transferase, Mn superoxide dismutase, C-reactive protein, Fibrinogen, Serum amyloid A, Metallothioneins, Ceruloplasmin, Lysozyme, Xanthine dehydrogenase, Xanthine oxidase, Platelet derived growth factor A chain (PDGF), Melanoma growth stimulatory activity (*gro-a,b,g*), Insulin-like growth factor-1 (IGF-1), Activin A, Pro-opiomelanocortiotropin, corticotropin releasing factor, B amyloid precursor, Basement membrane protein-40, Laminin B1 and B2, Constitutive heat shock protein p70, P42 mitogen, activating protein kinase, ornithine decarboxylase, heme oxygenase and G-protein a subunit). Other preferred antagonists include: hymenialdisine, herbimycines (e.g. herbamycin A), CK-103A and its derivatives (e.g. 4,6-dihydropyridazino[4,5-c]pyridazin-5 (1H)-one), CK-119, CK-122, iodomethacin, aflatoxin B1, leptin, heparin, bicyclic imidazoles (e.g SB203580), PD15306 HCl, podocarpic acid derivatives, M-20, Human [Gly2] Glucagon-like peptide-2, FR167653, Steroid derivatives, glucocorticoids, Quercetin, Theophylline, NO-synthetase inhibitors, RWJ 68354, Euclyptol (1.8-cineole), Magnosalin, N-Acetylcysteine, Alpha-Melatonin-Stimulating Hormone (a-MSH), Triclosan (2,4,4'-trichloro-2'-hydroxyldiphenyl ether), Prostaglandin E2 and 4-aminopyridine Ethacrynic acid and 4,4'-diisothiocyanatostilbene-2,2'-disulfonic acid (DIDS), Glucose, Lipophosphoglycan, aspirin, Catabolism-blocking agents, Diacerhein, Thiol-modulating agents, Zinc, Morphine, Leukotriene biosynthesis inhibitors (e.g. MK886), Platelet-activating factor receptor antagonists (e.g. WEB 2086), Amiodarone, Tranilast, S-methyl-L-thiocitrulline, Beta-adrenoreceptor agonists (e.g.Procaterol, Clenbuterol, Fenoterol, Terbutaline, Hyaluronic acid, anti-TNF-a antibodies, anti-IL-1a autoantibodies, IL-1 receptor antagonist, IL-1R- associated kinase, soluble TNF receptors and antiinflammatory cytokines (e.g IL-4, IL-13, IL-10, IL-6, TGF-b, angiotensin II, Soluble IL-1 type II receptor, Soluble IL-1 type I receptor, Tissue plasminogen activator, Zinc finger protein A20 IL-1 Peptides (e.g (Thr-Lys-Pro-Arg) (Tuftsin), (Ile-Thr-Gly-Ser-Glu) IL-1-alpha, Val-Thr-Lys-Phe-Tyr-Phe, Val-Thr-Asp-Phe-Tyr-Phe, Interferon alpha2b, Interferon beta, IL-1-beta analogues (e.g. IL-1-beta tripeptide: Lys-D-Pro-Thr), glycosylated IL-1-alpha, and IL-1ra peptides.

The terms "IL-1 gene cluster" and "IL-1 loci" as used herein include all the nucleic acid at or near the 2q13 region of chromosome 2, including at least the IL-1A, IL-1B and IL-1RN genes and any other linked sequences. (Nicklin et al., Genomics 19: 382-84, 1994). The terms "IL-1A", "IL-1B", and "IL-1RN" as used herein refer to the genes coding for IL-1α, IL-1β, and IL-1 receptor antagonist, respectively. The gene accession number for IL-1A, IL-1B, and IL-1RN are X03833, X04500, and X64532, respectively.

"IL-1 functional mutation" refers to a mutation within the IL-1 gene cluster that results in an altered phenotype (i.e. affects the function of an IL-1 gene or protein). Examples include: IL-1A(+4845) allele 2, IL-1B (+3954) allele 2, IL-1B (-511) allele 1 and IL-1RN (+2018) allele 1.

"IL-1X (Z) allele Y " refers to a particular allelic form, designated Y, occurring at an IL-1 locus polymorphic site in gene X, wherein X is IL-1A, B, or RN or some other gene in the IL-1 gene loci, and positioned at or near nucleotide Z, wherein nucleotide Z is numbered relative to the major transcriptional start site, which is nucleotide +1, of the particular IL-1 gene X. As further used herein, the term "IL-1X allele (Z)" refers to all alleles of an IL-1 polymorphic site in gene X positioned at or near nucleotide Z. For example, the term "IL-1RN (+2018) allele" refers to alternative forms of the IL-1RN gene at marker +2018. "IL-1RN (+2018) allele 1" refers to a form of the IL-1RN gene which contains a cytosine (C) at position +2018 of the sense strand. Clay et al., Hum. Genet. 97:723-26, 1996. "IL-1RN (+2018) allele 2" refers to a form of the IL-1RN gene which contains a thymine (T) at position +2018 of the plus strand. When a subject has two identical IL-1RN alleles, the subject is said to be homozygous, or to - have the homozygous state. When a subject has two different IL-1RN alleles, the subject is said to be heterozygous, or to have the heterozygous state. The term "IL-1RN (+2018) allele 2,2" refers to the homozygous IL-1 RN (+2018) allele 2 state. Conversely, the term "IL-1RN (+2018) allele 1,1" refers to the homozygous IL-1 RN (+2018) allele 1 state. The term "IL-1RN (+2018) allele 1,2" refers to the heterozygous allele 1 and 2 state.

"IL-1 related" as used herein is meant to include all genes related to the human IL-1 locus genes on human chromosome 2 (2q 12-14). These include IL-1 genes of the human IL-1 gene cluster located at chromosome 2 (2q 13-14) which include: the IL-1A gene which encodes interleukin-1α, the IL-1B gene which encodes interleukin-1β, and the IL-1RN (or IL-1ra) gene which encodes the interleukin-1 receptor antagonist. Furthermore these IL-1 related genes include the type I and type II human IL-1 receptor genes located on human chromosome 2 (2q12) and their mouse homologs located on mouse chromosome 1 at position 19.5 cM. Interleukin-1α, interleukin-1β, and interleukin-1RN are related in so much as they all bind to IL-1 type I receptors, however only interleukin-1α and interleukin-1β are agonist ligands which activate IL-1 type I receptors, while interleukin-1RN is a naturally occurring antagonist ligand. Where the term "IL-1" is used in reference to a gene product or polypeptide, it is meant to refer to all gene products encoded by the interleukin-1 locus on human chromosome 2 (2q 12-14) and their corresponding homologs from other species or functional variants thereof. The term IL-1 thus includes secreted polypeptides which promote an inflammatory response, such as IL-1α and IL-1β, as well as a secreted polypeptide which antagonize inflammatory responses, such as IL-1 receptor antagonist and the IL-1 type II (decoy) receptor.

An "IL-1 receptor" or "IL-1R" refers to various cell membrane bound protein receptors capable of binding to and/or transducing a signal from IL-1 locus-encoded ligand. The term applies to any of the proteins which are capable of binding interleukin-1 (IL-1) molecules and, in their native configuration as mammalian plasma membrane proteins, presumably play a role in transducing the signal provided by IL-1 to a cell. As used herein, the term includes analogs of native proteins with IL-1-binding or signal transducing activity. Examples include the human and murine IL-1 receptors described in U.S. Patent No. 4,968,607. The term "IL-1 nucleic acid" refers to a nucleic acid encoding an IL-1 protein.

An "IL-1 polypeptide" and "IL-1 protein" are intended to encompass polypeptides comprising the amino acid sequence encoded by the IL-1 genomic DNA sequences shown in Figures 1, 2, and 3, or fragments thereof, and homologs thereof and include agonist and antagonist polypeptides.

"Increased risk" refers to a statistically higher frequency of occurrence of the disease or disorder in an individual in comparison to the frequency of occurrence of the disease or disorder in a population. A factor identified to be associated with increased risk is termed a "risk factor." Carrying a particular polymorphic allele is a risk factor for a particular cardiovascular disease, and is associated with an increased risk of the particular disease.

The term "interact" as used herein is meant to include detectable relationships or associations (e.g. biochemical interactions) between molecules, such as interactions between protein-protein, protein-nucleic acid, nucleic acid-nucleic acid and protein-small molecule or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. For example, an isolated nucleic acid encoding one of the subject IL-1 polypeptides preferably includes no more than 10 kilobases (kb) of nucleic acid sequence which naturally immediately flanks the IL-1 gene in genomic DNA, more preferably no more than 5kb of such naturally occurring flanking sequences, and most preferably less than 1.5kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

A "knock-in" transgenic animal refers to an animal that has had a modified gene introduced into its genome and the modified gene can be of exogenous or endogenous origin.

A "knock-out" transgenic animal refers to an animal in which there is partial or complete suppression of the expression of an endogenous gene (e.g, based on deletion of at least a portion of the gene, replacement of at least a portion of the gene with a second sequence, introduction of stop codons, the mutation of bases encoding critical amino acids, or the removal of an intron junction, etc.).

A "knock-out construct" refers to a nucleic acid sequence that can be used to decrease or suppress expression of a protein encoded by endogenous DNA sequences in a cell. In a simple example, the knock-out construct is comprised of a gene, such as the IL-1RN gene, with a deletion in a critical portion of the gene so that active protein cannot be expressed therefrom. Alternatively, a number of termination codons can be added to the native gene to cause early termination of the protein or an intron junction can be inactivated. In a typical knock-out construct, some portion of the gene is replaced with a selectable marker (such as the neo gene) so that the gene can be represented as follows: IL-1RN 5'/neo/ IL-1RN 3', where IL-1RN5' and IL-1RN 3', refer to genomic or cDNA sequences which are, respectively, upstream and downstream relative to a portion of the IL-1RN gene and where neo refers to a neomycin resistance gene. In another knock-out construct, a second selectable marker is added in a flanking position so that the gene can be represented as: IL-1RN/neo/IL-1RN/TK, where TK is a thymidine kinase gene which can be added to either the IL-1RN5' or the IL-1RN3' sequence of the preceding construct and which further can be selected against (i.e. is a negative selectable marker) in appropriate media. This two-marker construct allows the selection of homologous recombination events, which removes the flanking TK marker, from non-homologous recombination events which typically retain the TK sequences. The gene deletion and/or replacement can be from the exons, introns, especially intron junctions, and/or the regulatory regions such as promoters.

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern. An example of linkage disequilibrium is that which occurs between the alleles at the IL-1RN (+2018) and IL-1RN (VNTR) polymorphic sites. The two alleles at IL-1RN (+2018) are 100% in linkage disequilibrium with the two most frequent alleles of IL-1RN (VNTR), which are allele 1 and allele 2.

The term "marker" refers to a sequence in the genome that is known to vary among individuals. For example, the IL-1RN gene has a marker that consists of a variable number of tandem repeats (VNTR).

"Modulate" refers to the ability of a substance to regulate bioactivity. When applied to an IL-1 bioactivity, an agonist or antagonist can modulate bioactivity for example by agonizing or antagonizing an IL-1 synthesis, receptor interaction, or IL-1 mediated signal transduction mechanism.

A "mutated gene" or "mutation" or "functional mutation" refers to an allelic form of a gene, which is capable of altering the phenotype of a subject having the mutated gene relative to a subject which does not have the mutated gene. The altered phenotype caused by a mutation can be corrected or compensated for by certain agents. If a subject must be homozygous for this mutation to have an altered phenotype, the mutation is said to be recessive. If one copy of the mutated gene is sufficient to alter the phenotype of the subject, the mutation is said to be dominant. If a subject has one copy of the mutated gene and has a phenotype that is intermediate between that of a homozygous and that of a heterozygous subject (for that gene), the mutation is said to be co-dominant.

A "non-human animal" of the invention includes mammals such as rodents, non-human primates, sheep, dogs, cows, goats, etc. amphibians, such as members of the *Xenopus* genus, and transgenic avians (e.g. chickens, birds, etc.). The term "chimeric animal" is used herein to refer to animals in which the recombinant gene is found, or in which the recombinant gene is expressed in some but not all cells of the animal. The term "tissue-specific chimeric animal" indicates that one of the recombinant IL-1 genes is present and/or expressed or disrupted in some tissues but not-others. The term "non-human mammal" refers to any member of the class Mammalia, except for humans.

As used herein, the term "nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g. peptide nucleic acids) and as applicable to the embodiment being described, single (sense or antisense) and double-stranded polynucleotides.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long.

The term "propensity to disease," also "predisposition" or "susceptibility" to disease or any similar phrase, means that certain alleles are hereby discovered to be associated with or predictive of a subject's incidence of developing a particular disease (herein, a cardiovascular disease). The alleles are thus over-represented in frequency in individuals with disease as compared to healthy individuals. Thus, these alleles can be used to predict disease even in pre-symptomatic or pre-diseased individuals. These alleles are understood to relate to the disorder underlying the disease.

A "risk factor" is a factor identified to be associated with an increased risk of developing sepsis or for prognosticating on the rapidity and/or ultimate progression of sepsis in a subject.

"Sepsis", "septicemia", "septic syndrome" and "septic response" refers to those biochemical and physiological responses that have been identified as systemic manifestations of uncontrolled infection. Sepsis is a nonspecific response of the host to any of a variety of factors, including: 1) disseminated microorganisms or their biochemical products from a nidus of infection, 2) microorganisms or their biochemical products without an infected primary source and 3) local inflammatory mediators from an infectious source or from a sterile site without the participation of microorganisms or their biochemical products. Organisms commonly involved in provoking sepsis include Gram positive bacteria, Gram negative bacteria and fungi. As examples, strep and staph species can produce sepsis either from a local focus of infection (a pneumonia or a meningitis, for example) or from a systemic insult (as in toxic shock syndrome). As a further example, sepsis is a well-known concomitant of a genitourinary infection, so-called urosepsis, where the infecting agent is usually a Gram negative organism. *Neisseria meningiditis* may occasion a fulminant, rapidly progressive sepsis accompanying a meningitis. Gram negative organisms commonly produce endotoxin, understood to be bacterial wall lipopolysaccharides that can mediate the responses of sepsis. When endotoxin is involved in sepsis, a key event in the evolution of the syndrome is the activation of the mononuclear phagocyte system, with consequent release of IL-1 and TNF-alpha. Sepsis responses are also understood to follow non-infectious events such as acute pancreatitis. Similar biological events are understood to be able to lead to the septic response following an infectious or a non-infectious insult.

The physiological and biochemical responses characterizing sepsis include: 1) hyperdynamic cardiac parameters, 2) a reduced peripheral vascular resistance, 3) a narrowed arteriovenous oxygen difference and 4) elevated serum lactate levels. There is thought to be a continuum of sepsis that, if uncorrected, may lead to death. The stages of sepsis are understood to be similar to the stages for other types of shock. Shock may result from any serious assault on the body's homeostatic mechanisms, whether from hemorrhage, trauma, burn injury, myocardial infarction or sepsis. Shock consists of widespread hypoperfusion at the tissue level, due to reduction of blood volume, reduction of cardiac output or redistribution of effective circulation. This results in insufficient delivery of oxygen and metabolites to the cells and inadequate clearance of metabolic byproducts. The resultant shift from aerobic to anaerobic cellular metabolism leads to the accumulation of lactic acid in the tissues. The derangements accompanying shock are usually correctable at the outset, but progress to irreversible injury and cellular death.

Stages of sepsis similarly have early reversible changes and later irreversible changes. A staging system has been established to evaluate the extent of the patient's septic deterioration. During the initial, nonprogressive phase, Siegel's Stage A, compensatory mechanisms are activated and vital organ perfusion is maintained. With prolonged insult, this set of responses becomes exaggerated, with clinical symptoms of peripheral vasodilatation combined with decreased perfusion to vital organs. This is Siegel's Stage B. Hypotension may be a consequence of this decreased perfusion. Diminished organ perfusion leads to irreversible injury to vital systems, including the liver, kidney and respiratory failures characteristic of multiple organ failure. The lung has a particular vulnerability to the changes seen in sepsis. A pattern - of respiratory failure known as acute respiratory distress syndrome (ARDS) may accompany sepsis as well as other types of shock. As sepsis progresses, there is an increasing cycle of metabolic and circulatory abnormalities until frank shock sets in, as defined herein. Septic shock corresponds to Siegel's Stage C. Progression of shock combined with progression of sepsis leads to the preterminal condition of sepsis-related low output failure, Siegel's Stage D. A patient who has entered this state is considered to have sustained irreversible injury. Survival is not anticipated. "Neonate sepsis" refers to the set of septic responses, as defined herein, manifest in the newborn infant or the fetus. Neonatal infections may be characterized according to their timing: early neonatal infections tend to occur within several days of birth, while late onset neonatal sepsis becomes manifest after a latent period. Infections with Group B strep or with *Escherichia coli* tend to develop symptoms including sepsis, pneumonia and/or meningitis within four or five days of birth. Infections with Listeria or Candida, by contrast, are later in onset. Infections in infants are termed neonatal infections through the first few months of life. Later infections in infants under age two may also give rise to sepsis. The evaluation of a high fever in this population typically requires vigorous diagnostic interventions to identify a source. A fever of unknown origin (where no source can be found) may be treated aggressively with antibiotics until the possibility of bacteremia can be conclusively ruled out with blood cultures.

"Small molecule" as used herein, is meant to refer to a composition, which has a molecular weight of less than about 5kD and most preferably less than about 4kD. Small molecules can be nucleic acids, peptides, peptidomimetics, carbohydrates, lipids or other organic or inorganic molecules.

As used herein, the term "specifically hybridizes" or "specifically detects" refers to the ability of a nucleic acid molecule to hybridize to at least approximately 6 consecutive nucleotides of a sample nucleic acid.

"Transcriptional regulatory sequence" is a generic term used throughout the specification to refer to DNA sequences, such as initiation signals, enhancers, and promoters, which induce or control transcription of protein coding sequences with which they are operably linked.

As used herein, the term "transgene" means a nucleic acid sequence (encoding, e.g., one of the IL-1 polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of an IL-1 polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques. The term is intended to include all progeny generations. Thus, the founder animal and all F1, F2, F3, and so on, progeny thereof are included.

The term "treating" as used herein is intended to encompass curing as well as ameliorating at least one symptom of sepsis or at least one abnormality associated with sepsis.

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the - form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### 3.2 Predictive Medicine

### 3.2.1. Prognostic Assays and Kits

Based in part on the findings described in detail in the following examples, that patients having allele 2 of IL-1B (-511) are more likely to die from sepsis and from the findings that certain markers comprise haplotype patterns in individuals, the present invention provides methods and kits for determining whether a subject has or is likely to develop sepsis and/or the likely rate or extent of progression of the septicemia.

Specifically, Applicants have determined identified the following three haplotype patterns defined by four polymorphic loci in the IL-1 gene cluster as shown below.

| Haplotypes | IL-1A (+4845) | IL-1B (+3954) | IL-1B (-511) | IL-1RN (+2018) |
|---|---|---|---|---|
| Pattern 1 | Allele 2 | Allele 2 | Allele 1 | Allele 1 |
| Pattern 2 | Allele 1 | Allele 1 | Allele 2 | Allele 2 |
| Pattern 3 | Allele 1 | Allele 1 | Allele 1 | Allele 1 |

The "Pattern 2 (44112332) haplotype" comprises at least the following markers:

| |
|---|
| allele 4 of the 222/223 marker of IL-1A |
| allele 4 of the gz5/gz6 marker of IL-1A |
| allele 1 of the -889 marker of IL-1A |
| allele 1 of the +3954 marker of IL-1B |
| allele 2 of the -511 marker of IL-1B |
| allele 3 of the gaat.p33330 marker |
| allele 3 of the Y31 marker |
| allele 2 of the VNTR |

| |
|---|
| allele 2 of the +2018 marker of IL-1RN allele 1 of the +4845 marker of IL-1A |

In addition to the markers pointed out above, pattern 2 includes markers that are in linkage disequilibrium with the markers set forth above. For example, allele 2 of the IL-1RN (+2018) polymorphism (Clay et al. (1996) Hum Genet 97: 723-26), also referred to as exon 2 (8006) (GenBank:X64532 at 8006) is known to be in linkage disequilibrium with allele 2 of the IL-1RN (VNTR) polymorphic locus, which in turn is a part of the 44112332 human haplotype. Thus, allele 2 of the IL-1RN (+2018) locus (i.e. C at +2018), is an allelic variant associated with the 44112332 haplotype and therefore provides an alternative target for prognostic genotyping analysis to determine an individual's likelihood of developing a vascular disorder. Similarly, three other polymorphisms in an IL-1RN alternative exon (Exon lic, which produces an intracellular form of the gene product) are also in linkage disequilibrium with allele 2 of IL-1RN (VNTR) (Clay et al. (1996) Hum Genet 97: 723-26). These include: the IL-1RN exon lic (1812) polymorphism (GenBank:X77090 at 1812); the IL-1RN exon lic (1868) polymorphism (GenBank:X77090 at 1868); and the IL-1RN exon 1ic (1887) polymorphism (GenBank:X77090 at 1887). Furthermore yet another polymorphism in the promoter for the alternatively spliced intracellular form of the gene, the Pic (1731) polymorphism (GenBank:X77090 at 1731), is also in linkage disequilibrium with allele 2 of the IL-1RN (VNTR) polymorphic locus (Clay et al. (1996) Hum Genet 97: 723-26). The corresponding sequence alterations for each of these IL-1RN polymorphic loci is shown below.

| Allele # | Exon 2 (+2018 of IL-1RN) | Exon 1ic -1 (1812 of GB: X77090) | Exon 1ic -2 (1868 of GB: X77090 | Exon 1ic -3 (1887 of GB:X77090) | Pic (1731 of GB: X77090) |
|---|---|---|---|---|---|
| 1 | T | G | A | G | G |
| 2 | C | A | G | C | A |

For each of these polymorphic loci, the allele 2 sequence variant has been determined to be in linkage disequilibrium with allele 2 of the IL-1RN (VNTR) locus (Clay et al. (1996) Hum Genet 97: 723-26). The pattern 2 haplotype appears to be associated with decreased levels of IL-1 receptor antagonist.

In addition to the allelic patterns described above, as described herein, one of skill in the art can readily identify other alleles (including polymorphisms and mutations) that are in linkage disequilibrium with a pattern 2 marker. For example, one could genotype a large population and perform statistical analysis to determine which alleles appear more commonly together than expected. Preferably the group is chosen to be comprised of genetically related individuals. Genetically related individuals include individuals from the same race, the same ethnic group, or even the same family. As the degree of genetic relatedness between a control group and a test group increases, so does the predictive value of polymorphic alleles which are ever more distantly linked to a disease-causing allele. This is because less evolutionary time has passed to allow polymorphisms which are linked along a chromosome in a founder population to redistribute through genetic cross-over events. Thus race-specific, ethnic-specific, and even family-specific diagnostic genotyping assays can be developed to allow for the detection of disease alleles which arose at ever more recent times in human evolution, e.g., after divergence of the major human races, after the separation of human populations into distinct ethnic groups, and even within the recent history of a particular family line.

Linkage disequilibrium between two polymorphic markers or between one polymorphic marker and a disease-causing mutation is a meta-stable state. Absent selective pressure or the sporadic linked reoccurrence of the underlying mutational events, the polymorphisms will eventually become disassociated by chromosomal recombination events and will thereby reach linkage equilibrium through the course of human evolution. Thus, the likelihood of finding a polymorphic allele in linkage disequilibrium with a disease or condition may increase with changes in at least two factors: decreasing physical distance between the polymorphic marker and the disease-causing mutation, and decreasing number of meiotic generations available for the dissociation of the linked pair. Consideration of the latter factor suggests that, the more closely related two individuals are, the more likely they will share a common parental chromosome or chromosomal region containing the linked polymorphisms and the less likely that this linked pair will have become unlinked through meiotic cross-over events occurring each generation. As a result, the more closely related two individuals are, the more likely it is that widely spaced polymorphisms may be co-inherited. Thus, for individuals related by common race, ethnicity or family, the reliability of ever more distantly spaced polymorphic loci can be relied upon as an indicator of inheritance of a linked disease-causing mutation.

The oligonucleotides present in one embodiment of a kit according to the present invention may be used for amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization). Examples of appropriate primers for use in the above described detection methods, include:
5'-CTCAGCAACACTCCTAT-3' (SEQ ID NO.1);
5'-TCCTGGTCTGCAGGTAA-3' (SEQ ID NO. 2);
which can be used to amplify and type the human IL-1RN (VNTR) polymorphic locus;
5'-CTA TCT GAG GAA CAA CCA ACT AGT AGC-3' (SEQ ID NO. 3);
5'-TAG GAC ATT GCA CCT AGG GTT TGT -3' (SEQ ID NO. 4);
which can be used to amplify and type the human IL-1RN (+2018) polymorphic locus;
5' TGGCATTGATCTGGTTCATC 3' (SEQ ID No: 5);
5' GTTTAGGAATCTTCCCACTT 3' (SEQ ID No: 6);
which can be used to amplify and type the human IL-1B (-511) polymorphic locus.;
5' CTC AGG TGT CCT CGA AGA AAT CAA A 3' (SEQ ID NO:7);
5' GCT TTT TTG CTG TGA GTC CCG 3' (SEQ ID NO:8)
which can be used to amplify and type the human IL-1B (+3954) polymorphic locus; and
5' ATG GTT TTA GAA ATC ATC AAG CCT AGG GCA 3' (SEQ ID NO:9)
3' AAT GAA AGG AGG GGA GGA TGA CAG AAA TGT 3' (SEQ ID NO:10)
which can be used to amplify and type the human IL-1A (+4845) polymorphic locus.

Appropriate probes may be designed to hybridize to a specific gene of the-IL-1 locus, such as IL-1A, IL-1B or IL-1RN or a related gene. Alternatively, these probes may incorporate other regions of the relevant genomic locus, including intergenic sequences. Indeed the IL-1 region of human chromosome 2 spans some 400,000 base pairs and, assuming an average of one single nucleotide polymorphism every 1,000 base pairs, includes some 400 SNPs loci alone. Yet other polymorphisms available for use with the immediate invention are obtainable from various public sources. For example, the human genome database collects intragenic SNPs, is searchable by sequence and currently contains approximately 2,700 entries (http://hgbase.interactiva.de). Also available is a human polymorphism database maintained by the Massachusetts Institute of Technology (MIT SNP database (http://www.genome.wi.mit.edu/SNP/human/index.html)). From such sources SNPs as well as other human polymorphisms may be found.

For example, examination of the IL-1 region of the human genome in any one of these databases reveals that the IL-1 locus genes are flanked by a centromere proximal polymorphic marker designated microsatellite marker AFM220ze3 at 127.4 cM (centiMorgans) (see GenBank Acc. No. Z17008) and a distal polymorphic marker designated microsatellite anchor marker AFM087xa1 at 127.9 cM (see GenBank Acc. No. Z16545). These human polymorphic loci are both CA dinucleotide repeat microsatellite polymorphisms, and, as such, show a high degree of heterozygosity in human populations. For example, one allele of AFM220ze3 generates a 211 bp PCR amplification product with a 5' primer of the sequence TGTACCTAAGCCCACCCTTTAGAGC (SEQ ID No.11) and a 3' primer of the sequence TGGCCTCCAGAAACCTCCAA (SEQ ID No.12). Furthermore, one allele of AFM087xa1 generates a 177 bp PCR amplification product with a 5' primer of the sequence GCTGATATTCTGGTGGGAAA (SEQ ID No.13) and a 3' primer of the sequence GGCAAGAGCAAAACTCTGTC (SEQ ID No. 14 ). Equivalent primers corresponding to unique sequences occurring 5' and 3' to these human chromosome 2 CA dinucleotide repeat polymorphisms will be apparent to one of skill in the art. Reasonable equivalent primers include those which hybridize within about 1 kb of the designated primer, and which further are anywhere from about 17 bp to about 27 bp in length. A general guideline for designing primers for amplification of unique human chromosomal genomic sequences is that they possess a melting temperature of at least about 50°C, wherein an approximate melting temperature can be estimated using the formula Tₘₑₗₜ = [2 x (# of A or T) + 4 x (# of G or C)].

A number of other human polymorphic loci occur between these two CA dinucleotide repeat polymorphisms and provide additional targets for determination of a cardio-vascular disorder prognostic allele in a family or other group of genetically related individuals. For example, the National Center for Biotechnology Information web site (www.ncbi.nlm.nih.gov/genemap/) lists a number of polymorphism markers in the region of the IL-1 locus and provides guidance in designing appropriate primers for amplification and analysis of these markers.

Accordingly, the nucleotide segments of the invention may be used for their ability to selectively form duplex molecules with complementary stretches of human chromosome 2 q 12-13 or cDNAs from that region or to provide primers for amplification of DNA or cDNA from this region. The design of appropriate probes for this purpose requires consideration of a number of factors. For example, fragments having a length of between 10, 15, or 18 nucleotides to about 20, or to about 30 nucleotides, will find particular utility. Longer sequences, e.g., 40, 50, 80, 90, 100, even up to full length, are even more preferred for certain embodiments. Lengths of oligonucleotides of at least about 18 to 20 nucleotides are well accepted by those of skill in the art as sufficient to allow sufficiently specific hybridization so as to be useful as a molecular probe. Furthermore, depending on the application envisioned, one will desire to employ varying conditions of hybridization to achieve varying degrees of selectivity of probe towards target sequence. For applications requiring high selectivity, one will typically desire to employ relatively stringent conditions to form the hybrids. For example, relatively low salt and/or high temperature conditions, such as provided by 0.02 M-0.15M NaCl at temperatures of about 50° C to about 70° C. Such selective conditions may tolerate little, if any, mismatch between the probe and the template or target strand.

In one embodiment, the method comprises genotyping a nucleic acid sample obtained from the subject to determine at least one allele of an IL-1 gene that leads to a dysregulated inflammatory response. Such an allele can be detected, for example, by determining the transcription rate or mRNA and/or protein level of an IL-1 gene or protein, such as by Northern blot analysis, reverse transcription-polymerase chain reaction (RT-PCR), *in situ* hybridization, immunoprecipitation, Western blot hybridization, or immunohistochemistry. According to one method, cells are obtained from a subject and the IL-1 protein or mRNA level is determined and compared to the level of IL-1 protein or mRNA level in a subject that is not susceptible to the development of sepsis or is not likely to progress rapidly into septic shock.

In another embodiment, the method comprises measuring at least one activity of an IL-1 protein. For example, the constant of affinity of an IL-1 a or β protein of a subject with a receptor can be determined. The results obtained can then be compared with results from the same analysis performed on a subject, who is known to be susceptible to sepsis or is known to not be susceptible to the development of sepsis.

In preferred embodiments, the method is characterized as comprising genotyping a nucleic acid sample obtained from the subject to determine at least one allele of an IL-1 genetic pattern that leads to a dysregulated inflammatory response. In an exemplary embodiment, there is provided a nucleic acid composition comprising a nucleic acid probe including a region of nucleotide sequence which is capable of hybridizing to a sense or antisense sequence of at least one allele of an IL-1 genetic pattern that leads to a dysregulated inflammatory response. For example, the nucleic acid can be rendered accessible for hybridization, the probe contacted with the nucleic acid of the sample, and the hybridization of the probe to the sample nucleic acid detected. Such technique can be used to detect alterations or allelic variants at either the genomic or mRNA level as well as to determine mRNA transcript levels.

A preferred detection method is allele specific hybridization using probes overlapping a region of at least one allele of an IL-1 genetic pattern that leads to a dysregulated inflammatory response and having about 5, 10, 20, 25, or 30 nucleotides around the mutation or polymorphic region. In a preferred embodiment of the invention, several probes capable of hybridizing specifically to other allelic variants involved in sepsis are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), and Q-Beta Replicase (Lizardi, P.M. et al., 1988, Bio/Technology 6:1197).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of an IL-1 genetic pattern that leads to a dysregulated inflammatory response under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of an IL-1 genetic pattern that leads to a dysregulated inflammatory response is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety of sequencing reactions known in the art can be used to directly sequence the allele. Exemplary sequencing reactions include those based on techniques developed by Maxim and Gilbert (Proc. Natl Acad Sci USA (1977) 74:560) or Sanger (Sanger et al (1977) Proc. Nat. Acad. Sci 74:5463). It is also contemplated that any of a variety of automated sequencing procedures may be utilized when performing the subject assays (*Biotechniques* (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one skilled in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labelled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; Saleeba et al (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of *E. coli* cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on IL-1β allele 1 (+6912) is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify the allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of the sample and control allele are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of alleles in polyacrylamide gels containing a gradient of denaturant is assayed using denaturing gradient gel electrophoresis (DGGE) (Myers et al (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238. In addition it may be desirable to introduce a novel restriction site in the region of the mutation to create cleavage-based detection (Gasparini et al (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al., Science 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., Proc. Natl. Acad. Sci. (U.S.A.) 87:8923-8927 (1990). In this method, PCR is used to achieve the exponential amplification of target DNA, which is then detected using OLA.

Several techniques based on this OLA method have been developed and can be used to detect alleles of an IL-1 genetic pattern that leads to a dysregulated inflammatory response. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA^{™} in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., Amer.J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-21; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential *in vitro* transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment the DNA sample is obtained from a bodily fluid, e.g, blood, obtained by known techniques (e.g. venipuncture) or saliva. Alternatively, nucleic acid tests can be performed on dry samples (e.g. hair or skin). When using RNA or protein, the cells or tissues that may be utilized must express the IL-1 gene.

Diagnostic procedures may also be performed *in situ* directly upon tissue sections (fixed and/or frozen) of patient tissue obtained from biopsies or resections, such that no nucleic acid purification is necessary. Nucleic acid reagents may be used as probes and/or primers for such *in situ* procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

Another embodiment of the invention is directed to kits for detecting a predisposition for developing sepsis and/or for progressing more rapidly or severely. This kit may contain one or more oligonucleotides, including 5' and 3' oligonucleotides that hybridize 5' and 3' to at least one allele of an IL-1 proinflammatory haplotype. PCR amplification oligonucleotides should hybridize between 25 and 2500 base pairs apart, preferably between about 100 and about 500 bases apart, in order to produce a PCR product of convenient size for subsequent analysis.

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

The kit may, optionally, also include DNA sampling means such as the AmpliCard™ (University of Sheffield, Sheffield, England S10 2JF; Tarlow, JW, et al., J. of Invest. Dermatol. 103:387-389 (1994)) and the like; DNA purification reagents such as Nucleon™ kits, lysis buffers, proteinase solutions and the like; PCR reagents, such as 10x reaction buffers, thermostable polymerase, dNTPs, and the like; and allele detection means such as the *Hinf*I restriction enzyme, allele specific oligonucleotides, degenerate oligonucleotide primers for nested PCR from dried blood.

### 3.2.2. Pharmacogenomics

Knowledge of the particular IL-1 polymorphisms that are predictive of sepsis, alone or in conjunction with information on other genetic defects contributing to sepsis (the genetic profile of sepsis) allows a customization of the therapy for a particular disease to the individual's genetic profile, the goal of "pharmacogenomics". For example, subjects carrying a pattern 2 allele may be predisposed to developing sepsis or for progressing more rapidly or severely into sepsis related sequelae. Thus, comparison of an individual's IL-1 genetic profile to the population profile for sepsis, permits the selection or design of drugs that are expected to be safe and efficacious for a particular patient or patient population (i.e., a group of patients having the same genetic alteration).

The ability to target populations expected to show the highest clinical benefit, based on the IL-1 genetic profile, can enable: 1) the repositioning of marketed sepsis drugs with disappointing market results; 2) the rescue of sepsis drug candidates whose clinical development has been discontinued as a result of safety or efficacy limitations, which are patient subgroup-specific; and 3) an accelerated and less costly development for sepsis drug candidates and more optimal drug labeling.

Cells of a subject may also be obtained before and after administration of a therapeutic to detect the level of expression of genes other than IL-1, to verify that the therapeutic does not increase or decrease the expression of genes which could be deleterious. This can be done, e.g., by using the method of transcriptional profiling. Thus, mRNA from cells exposed in vivo to a therapeutic and mRNA from the same type of cells that were not exposed to the therapeutic could be reverse transcribed and hybridized to a chip containing DNA from numerous genes, to thereby compare the expression of genes in cells treated and not treated with the therapeutic.

### 4.1 Sepsis Therapeutics

Modulators of IL-1 (e.g. IL-1α, IL-1β or IL-1 receptor antagonist) or a protein encoded by a gene that is in linkage disequilibrium with an IL-1 gene can comprise any type of compound, including a protein, peptide, peptidomimetic, small molecule, or nucleic acid. Preferred IL-1 agonists include nucleic acids (e.g. encoding an IL-1 protein or a gene that is up- or down-regulated by an IL-1 protein), proteins (e.g. IL-1 proteins or a protein that is up- or down-regulated thereby) or a small molecule (e.g. that regulates expression or binding of an IL-1 protein). Preferred IL-1 antagonists include nucleic acids (e.g. single (antisense) or double stranded (triplex) DNA or PNA and ribozymes), protein (e.g. antibodies) and small molecules that act to suppress or inhibit IL-1 transcription and/or protein activity.

### 4.1.1. Effective Dose

Toxicity and therapeutic efficacy of such compounds can be determined by standard pharmaceutical procedures in cell cultures or experimental animals, e.g., for determining The LD₅₀ (the dose lethal to 50% of the population) and the Ed₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissues in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### 4.1.2. Formulation and Use

Compositions for use in accordance with the present invention may be formulated in a conventional manner using one or more physiologically acceptable carriers or excipients. Thus, the compounds and their physiologically acceptable salts and solvates may be formulated for administration by, for example, injection, inhalation or insufflation (either through the mouth or the nose) or oral, buccal, parenteral or rectal administration.

For such therapy, the compounds of the invention can be formulated for a variety of loads of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous. For injection, the compounds of the invention can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner. For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt. Other suitable delivery systems include microspheres which offer the possibility of local noninvasive delivery of drugs over an extended period of time. This technology utilizes microspheres of precapillary size which can be injected via a coronary catheter into any selected part of the e.g. heart or other organs without causing inflammation or ischemia. The administered therapeutic is slowly released from these microspheres and taken up by surrounding tissue cells (e.g. endothelial cells).

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the oligomers of the invention are formulated into ointments, salves, gels, or creams as generally known in the art. A wash solution can be used locally to treat an injury or inflammation to accelerate healing.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 4.2 Assays to Identify Sepsis Therapeutics

Based on the identification of mutations that cause or contribute to the development of a vascular disorder, the invention further features cell-based or cell free assays, e.g., for identifying sepsis therapeutics. In one embodiment, a cell expressing an IL-1 receptor, or a receptor for a protein that is encoded by a gene which is in linkage disequilibrium with an IL-1 gene, on the outer surface of its cellular membrane is incubated in the presence of a test compound alone or in the presence of a test compound and another protein and the interaction between the test compound and the receptor or between the protein (preferably a tagged protein) and the receptor is detected, e.g., by using a microphysiometer (McConnell et al. (1992) Science 257:1906). An interaction between the receptor and either the test compound or the protein is detected by the microphysiometer as a change in the acidification of the medium. This assay system thus provides a means of identifying molecular antagonists which, for example, function by interfering with protein- receptor interactions, as well as molecular agonist which, for example, function by activating a receptor.

Cellular or cell-free assays can also be used to identify compounds which modulate expression of an IL-1 gene or a gene in linkage disequilibrium therewith, modulate translation of an mRNA, or which modulate the stability of an mRNA or protein. Accordingly, in one embodiment, a cell which is capable of producing an IL-1, or other protein is incubated with a test compound and the amount of protein produced in the cell medium is measured and compared to that produced from a cell which has not been contacted with the test compound. The specificity of the compound vis a vis the protein can be confirmed by various control analysis, e.g., measuring the expression of one or more control genes. In particular, this assay can be used to determine the efficacy of antisense, ribozyme and triplex compounds.

Cell-free assays can also be used to identify compounds which are capable of interacting with a protein, to thereby modify the activity of the protein. Such a compound can, e.g., modify the structure of a protein thereby effecting its ability to bind to a receptor. In a preferred embodiment, cell-free assays for identifying such compounds consist essentially in a reaction mixture containing a protein and a test compound or a library of test compounds in the presence or absence of a binding partner. A test compound can be, e.g., a derivative of a binding partner, e.g., a biologically inactive target peptide, or a small molecule.

Accordingly, one exemplary screening assay of the present invention includes the steps of contacting a protein or functional fragment thereof with a test compound or library of test compounds and detecting the formation of complexes. For detection purposes, the molecule can be labeled with a specific marker and the test compound or library of test compounds labeled with a different marker. Interaction of a test compound with a protein or fragment thereof can then be detected by determining the level of the two labels after an incubation step and a washing step. The presence of two labels after the washing step is indicative of an interaction.

An interaction between molecules can also be identified by using real-time BIA (Biomolecular Interaction Analysis, Pharmacia Biosensor AB) which detects surface plasmon resonance (SPR), an optical phenomenon. Detection depends on changes in the mass concentration of macromolecules at the biospecific interface, and does not require any labeling of interactants. In one embodiment, a library of test compounds can be immobilized on a sensor surface, e.g., which forms one wall of a micro-flow cell. A solution containing the protein or functional fragment thereof is then flown continuously over the sensor surface. A change in the resonance angle as shown on a signal recording, indicates that an interaction has occurred. This technique is further described, e.g., in BIAtechnology Handbook by Pharmacia.

Another exemplary screening assay of the present invention includes the steps of (a) forming a reaction mixture including: (i) an IL-1 or other protein, (ii) an appropriate receptor, and (iii) a test compound; and (b) detecting interaction of the protein and receptor. A statistically significant change (potentiation or inhibition) in the interaction of the protein and receptor in the presence of the test compound, relative to the interaction in the absence of the test compound, indicates a potential antagonist (inhibitor). The compounds of this assay can be contacted simultaneously. Alternatively, a protein can first be contacted with a test compound for an appropriate amount of time, following which the receptor is added to the reaction mixture. The efficacy of the compound can be assessed by generating dose response curves from data obtained using various concentrations of the test compound. Moreover, a control assay can also be performed to provide a baseline for comparison.

Complex formation between a protein and receptor may be detected by a variety of techniques. Modulation of the formation of complexes can be quantitated using, for example, detectably labeled proteins such as radiolabeled, fluorescently labeled, or enzymatically labeled proteins or receptors, by immunoassay, or by chromatographic detection.

Typically, it will be desirable to immobilize either the protein or the receptor to facilitate separation of complexes from uncomplexed forms of one or both of the proteins, as well as to accommodate automation of the assay. Binding of protein and receptor can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase fusion proteins can be adsorbed onto glutathione sepharose beads (Sigma Chemical, St. Louis, MO) or glutathione derivatized microtitre plates, which are then combined with the receptor, e.g. an ³⁵S-labeled receptor, and the test compound, and the mixture incubated under conditions conducive to complex formation, e.g. at physiological conditions for salt and pH, though slightly more stringent conditions may be desired. Following incubation, the beads are washed to remove any unbound label, and the matrix immobilized and radiolabel determined directly (e.g. beads placed in scintillant), or in the supernatant after the complexes are subsequently dissociated. Alternatively, the complexes can be dissociated from the matrix, separated by SDS-PAGE, and the level of protein or receptor found in the bead fraction quantitated from the gel using standard electrophoretic techniques such as described in the appended examples. Other techniques for immobilizing proteins on matrices are also available for use in the subject assay. For instance, either protein or receptor can be immobilized utilizing conjugation of biotin and streptavidin. Transgenic animals can also be made to identify agonists and antagonists or to confirm the safety and efficacy of a candidate therapeutic. Transgenic animals of the invention can include non-human animals containing a sepsis causative mutation under the control of an appropriate endogenous promoter or under the control of a heterologous promoter.

The transgenic animals can also be animals containing a transgene, such as reporter gene, under the control of an appropriate promoter or fragment thereof. These animals are useful, e.g., for identifying drugs that modulate the activity of an IL-1 protein, such as by modulating gene expression. Methods for obtaining transgenic non-human animals are well known in the art. In preferred embodiments, the expression of the sepsis causative mutation is restricted to specific subsets of cells, tissues or developmental stages utilizing, for example, cis-acting sequences that control expression in the desired pattern. In the present invention, such mosaic expression of a protein can be essential for many forms of lineage analysis and can additionally provide a means to assess the effects of, for example, expression level which might grossly alter development in small patches of tissue within an otherwise normal embryo. Toward this end, tissue-specific regulatory sequences and conditional regulatory sequences can be used to control expression of the mutation in certain spatial patterns. Moreover, temporal patterns of expression can be provided by, for example, conditional recombination systems or prokaryotic transcriptional regulatory sequences. Genetic techniques, which allow for the expression of a mutation can be regulated via site-specific genetic manipulation *in vivo*, are known to those skilled in the art.

The transgenic animals of the present invention all include within a plurality of their cells a sepsis causative mutation transgene of the present invention, which transgene alters the phenotype of the "host cell". In an illustrative embodiment, either the *cre*/*loxP* recombinase system of bacteriophage P1 (Lakso et al. (1992) PNAS 89:6232-6236; Orban et al. (1992) PNAS 89:6861-6865) or the FLP recombinase system of *Saccharomyces cerevisiae* (O'Gorman et al. (1991) Science 251:1351-1355; PCT publication WO 92/15694) can be used to generate *in vivo* site-specific genetic recombination systems. Cre recombinase catalyzes the site-specific recombination of an intervening target sequence located between *loxP* sequences. *loxP* sequences are 34 base pair nucleotide repeat sequences to which the Cre recombinase binds and are required for Cre recombinase mediated genetic recombination. The orientation of *loxP* sequences determines whether the intervening target sequence is excised or inverted when Cre recombinase is present (Abremski et al. (1984) J. Biol. Chem. 259:1509-1514); catalyzing the excision of the target sequence when the *loxP* sequences are oriented as direct repeats and catalyzes inversion of the target sequence when *loxP* sequences are oriented as inverted repeats.

Accordingly, genetic recombination of the target sequence is dependent on expression of the Cre recombinase. Expression of the recombinase can be regulated by promoter elements which are subject to regulatory control, e.g., tissue-specific, developmental stage-specific, inducible or repressible by externally added agents. This regulated control will result in genetic recombination of the target sequence only in cells where recombinase expression is mediated by the promoter element. Thus, the activation of expression of the causative mutation transgene can be regulated via control of recombinase expression.

Use of the *crelloxP* recombinase system to regulate expression of a causative mutation transgene requires the construction of a transgenic animal containing transgenes encoding both the Cre recombinase and the subject protein. Animals containing both the Cre recombinase and the sepsis causative mutation transgene can be provided through the construction of "double" transgenic animals. A convenient method for providing such animals is to mate two transgenic animals each containing a transgene.

Similar conditional transgenes can be provided using prokaryotic promoter sequences which require prokaryotic proteins to be simultaneous expressed in order to facilitate expression of the transgene. Exemplary promoters and the corresponding trans-activating prokaryotic proteins are given in U.S. Patent No. 4,833,080.

Moreover, expression of the conditional transgenes can be induced by gene therapy-like methods wherein a gene encoding the transactivating protein, e.g. a recombinase or a prokaryotic protein, is delivered to the tissue and caused to be expressed, such as in a cell-type specific manner. By this method, the transgene could remain silent into adulthood until "turned on" by the introduction of the transactivator.

In an exemplary embodiment, the "transgenic non-human animals" of the invention are produced by introducing transgenes into the germline of the non-human animal. Embryonal target cells at various developmental stages can be used to introduce transgenes. Different methods are used depending on the stage of development of the embryonal target cell. The specific line(s) of any animal used to practice this invention are selected for general good health, good embryo yields, good pronuclear visibility in the embryo, and good reproductive fitness. In addition, the haplotype is a significant factor. For example, when transgenic mice are to be produced, strains such as C57BL/6 or FVB lines are often used (Jackson Laboratory, Bar Harbor, ME). Preferred strains are those with H-2^{b}, H-2^{d} or H-2^{q} haplotypes such as C57BL/6 or DNA/1. The line(s) used to practice this invention may themselves be transgenics, and/or may be knockouts (i.e., obtained from animals which have one or more genes partially or completely suppressed).

In one embodiment, the transgene construct is introduced into a single stage embryo. The zygote is the best target for microinjection. In the mouse, the male pronucleus reaches the size of approximately 20 micrometers in diameter which allows reproducible injection of 1-2 pl of DNA solution. The use of zygotes as a target for gene transfer has a major advantage in that in most cases the injected DNA will be incorporated into the host gene before the first cleavage (Brinster et al. (1985) PNAS 82:4438-4442). As a consequence, all cells of the transgenic animal will carry the incorporated transgene. This will in general also be reflected in the efficient transmission of the transgene to offspring of the founder since 50% of the germ cells will harbor the transgene.

Normally, fertilized embryos are incubated in suitable media until the pronuclei appear. At about this time, the nucleotide sequence comprising the transgene is introduced into the female or male pronucleus as described below. In some species such as mice, the male pronucleus is preferred. It is most preferred that the exogenous genetic material be added to the male DNA complement of the zygote prior to its being processed by the ovum nucleus or the zygote female pronucleus. It is thought that the ovum nucleus or female pronucleus release molecules which affect the male DNA complement, perhaps by replacing the protamines of the male DNA with histones, thereby facilitating the combination of the female and male DNA complements to form the diploid zygote. Thus, it is preferred that the exogenous genetic material be added to the male complement of DNA or any other complement of DNA prior to its being affected by the female pronucleus. For example, the exogenous genetic material is added to the early male pronucleus, as soon as possible after the formation of the male pronucleus, which is when the male and female pronuclei are well separated and both are located close to the cell membrane. Alternatively, the exogenous genetic material could be added to the nucleus of the sperm after it has been induced to undergo decondensation. Sperm containing the exogenous genetic material can then be added to the ovum or the decondensed sperm could be added to the ovum with the transgene constructs being added as soon as possible thereafter.

Introduction of the transgene nucleotide sequence into the embryo may be accomplished by any means known in the art such as, for example, microinjection, electroporation, or lipofection. Following introduction of the transgene nucleotide sequence into the embryo, the embryo may be incubated *in vitro* for varying amounts of time, or reimplanted into the surrogate host, or both. In vitro incubation to maturity is within the scope of this invention. One common method in to incubate the embryos in vitro for about 1-7 days, depending on the species, and then reimplant them into the surrogate host.

For the purposes of this invention a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes. Thus, the gamete nuclei must be ones which are naturally compatible, i.e., ones which result in a viable zygote capable of undergoing differentiation and developing into a functioning organism. Generally, a euploid zygote is preferred. If an aneuploid zygote is obtained, then the number of chromosomes should not vary by more than one with respect to the euploid number of the organism from which either gamete originated.

In addition to similar biological considerations, physical ones also govern the amount (e.g., volume) of exogenous genetic material which can be added to the nucleus of the zygote or to the genetic material which forms a part of the zygote nucleus. If no genetic material is removed, then the amount of exogenous genetic material which can be added is limited by the amount which will be absorbed without being physically disruptive. Generally, the volume of exogenous genetic material inserted will not exceed about 10 picoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs which are added to the zygote is dependent upon the total amount of exogenous genetic material added and will be the amount which enables the genetic transformation to occur. Theoretically only one copy is required; however, generally, numerous copies are utilized, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anesthetized, and the embryos are inserted into the oviduct. The number of embryos implanted into a particular host will vary by species, but will usually be comparable to the number of off spring the species naturally produces.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, DNA is prepared from tail tissue and analyzed by Southern analysis or PCR for the transgene. Alternatively, the tissues or cells believed to express the transgene at the highest levels are tested for the presence and expression of the transgene using Southern analysis or PCR, although any tissues or cell types may be used for this analysis.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by *in vitro* fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. Where *in vitro* fertilization is used, the fertilized embryo may be implanted into a surrogate host or incubated *in vitro,* or both. Using either method, the progeny may be evaluated for the presence of the transgene using methods described above, or other appropriate methods.

The transgenic animals produced in accordance with the present invention will include exogenous genetic material. Further, in such embodiments the sequence will be attached to a transcriptional control element, e.g., a promoter, which preferably allows the expression of the transgene product in a specific type of cell.

Retroviral infection can also be used to introduce the transgene into a non-human animal. The developing non-human embryo can be cultured *in vitro* to the blastocyst stage. During this time, the blastomeres can be targets for retroviral infection (Jaenich, R. (1976) PNAS 73:1260-1264). Efficient infection of the blastomeres is obtained by enzymatic treatment to remove the zona pellucida (Manipulating the Mouse Embryo, Hogan eds. (Cold Spring Harbor Laboratory Press, Cold Spring Harbor, 1986). The viral vector system used to introduce the transgene is typically a replication-defective retrovirus carrying the transgene (Jahner et al. (1985) PNAS 82:6927-6931; Van der Putten et al. (1985) PNAS 82:6148-6152). Transfection is easily and efficiently obtained by culturing the blastomeres on a monolayer of virus-producing cells (Van der Putten, *supra*; Stewart et al. (1987) EMBO J. 6:383-388). Alternatively, infection can be performed at a later stage. Virus or virus-producing cells can be injected into the blastocoele (Jahner et al. (1982) Nature 298:623-628). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line by intrauterine retroviral infection of the midgestation embryo (Jahner et al. (1982) *supra*).

A third type of target cell for transgene introduction is the embryonal stem cell (ES). ES cells are obtained from pre-implantation embryos cultured *in vitro* and fused with embryos (Evans et al. (1981) Nature 292:154-156; Bradley et al. (1984) Nature 309:255-258; Gossler et al. (1986) PNAS 83: 9065-9069; and Robertson et al. (1986) Nature 322:445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R. (1988) Science 240:1468-1474.

The present invention is further illustrated by the following examples which should not be construed as limiting in any way. The practice of the present invention will employ, unless otherwise indicated, conventional techniques that are within the skill of the art. Such techniques are explained fully in the literature. See, for example, Molecular Cloning A Laboratory Manual, (2nd ed., Sambrook, Fritsch and Maniatis, eds., Cold Spring Harbor Laboratory Press: 1989); DNA Cloning, Volumes I and II (D. N. Glover ed., 1985); Oligonucleotide Synthesis (M. J. Gait ed., 1984); U.S. Patent No. 4,683,195; U.S. Patent No. 4,683,202; and Nucleic Acid Hybridization (B. D. Hames & S. J. Higgins eds., 1984).

### EXAMPLE 1: A polymorphism of the Interleukin-1 Gene and Death in Meningococcal Disease

A case control study was performed on caucasian patients aged 4-70 years with documented meningococcal disease, of whom 31 died and 97 survived. DNA from these patients was genotyped for known polymorphisms in the IL-1A gene (at position -889), in the IL-1B gene (at positions -511 and +3954), and the IL-1RN gene intron 2 (+2018) and the TNF α gene (at position -308).

As shown in the following table 1, patients who were homozygous for allele 2 of IL-1B (-511) were more likely to die (odds ratio 5.10; 95% CI 1.61-16.17, P = 0.001). Carriage of a single copy of the -511 allele was not significantly associated with death. Polymorphisms with any of the other genes studied were not significantly associated with death.

| TABLE 1 | | | | | | | | | | |
|---|---|---|---|---|---|---|---|---|---|---|
| **Homozygote and Heterozygote frequencies of IL-1 and TNF polymorphisms** | | | | | | | | | | |
| | **IL-1A (+4845)** | | **IL-1B(+3964)** | | **IL-1B (-511)** | | **IL-IRA (+2018)** | | **TNF (-308)** | |
| | **Homozygote** | **Heterozygote** | **Homozygote** | **Heterozygote** | **Homozygote** | **Heterozygote** | **Homozygote** | **Heterozygote** | **Homozygote** | **Heterozygote** |
| **Fatal cases (n =31)** | **1** | **14** | **0** | **12** | **8** | **6** | **5** | **13** | **0** | **11** |
| | **(3.2%)** | **(45.2%)** | **(0%)** | **(38.7%)** | **(25.8%)** | **(19.4%)** | **(16.1%)** | **(41.9%)** | **(0%)** | **(35.5%)** |
| **Surviving cases** | **12** | **40** | **5** | **37** | **6** | **42** | **9** | **37** | **3** | **25** |
| **(n=97)** | **(12.4%)** | **(41.2%)** | **(5.2%)** | **(38.1%)** | **(6.2%)** | **(43.2%)** | **(9.3%)** | **(38.1%)** | **(3%)** | **(25.8%)** |

### EXAMPLE 2: Identification of IL-1B (-511)

This C/T single base variation in the IL-1 beta promoter was described in di Giovine, F.S. et al., (1992) Hum. Mol. Genet. 1: 450. The gene accession number is X04500.

MgCl₂ is used at 2.5 mM final and PCR primer (5'-TGGCATTGATCTGGTTCATC-3' (SEQ ID. No. 15) and 5'-GTTTAGGAATCTTCCCACTT-3') (SEQ ID. No. 16) at 1µM. Cycling is performed at [95°, 1min] µl; [95°, 1 min; 53°, 1 min; 72°, 1 min;] x35; [72, 5 min] µl; 4°C. Each PCR reaction is divided in two 25µl aliquots; one is added of 3 Units of *Ava I*, the other 3.7 Units of *Bsu* 36 1, in addition to 3 µl of the specific 10X restriction buffer. Incubation is at 37°C overnight. Electrophoresis is by PAGE 9%.

Interpretation The two enzymes cut respectively the two different alleles. Ava I will produce 190 + 144 for allele 1, while it does not cut allele 2 (304 bp). The restriction pattern obtained should be the inverse in the two aliquots (identifying homozygotes) or identical (heterozygotes). Frequencies in North British Caucasian population are 0.61 and 0.39. For 90% power at 0.05 level of significance in a similar genetic pool, 133 cases should be studied to detect 1.5 fold increase in frequency, or 505 for 0.1 absolute increase in frequency.

## Claims

1. A method for determining whether a subject is or is not susceptible to developing and/or to rapidly progress into sepsis, said method comprising the steps of:
a) obtaining a nucleic acid sample from the subject; and
b) detecting an IL-1 pattern 2 allele in said sample, wherein the IL-1 pattern 2 allele is selected from the group consisting of allele 1 of IL-1A (+4845) and allele 2 of IL-1B (-511), wherein detection of a pattern 2 allele indicates that the patient has an increased susceptibility to developing sepsis.

2. The method of claim 1, wherein the IL-1 pattern 2 allele is allele 1 of IL-1A (+4845).

3. The method of claim 1, wherein said detecting step is selected from the group consisting of:
a) allele specific oligonucleotide hybridization;
b) size analysis;
c) sequencing;
d) hybridization;
e) 5' nuclease digestion;
f) single-stranded conformation polymorphism;
g) allele specific hybridization;
h) primer specific extension; and
j) oligonucleotide ligation assay.

4. The method of claim 1, further comprising amplifying the nucleic acid sample.

5. The method of claim 3, wherein said size analysis is preceded by a restriction enzyme digestion.

6. The method of claim 1, wherein the IL-1 pattern 2 allele is IL-1B allele 2 (-511).

7. A method for selecting a therapeutic for the treatment or prevention of sepsis in a subject, comprising:
a) determining whether the subject has an IL-1 pattern 2 allele, wherein the IL-1 pattern 2 allele is selected from the group consisting of allele 1 of IL-IA (+4845) and allele 2 of IL-1B (-511); and
b) selecting an appropriate sepsis therapeutic.

8. The method of claim 7, wherein said detecting is performed using a technique selected from the group consisting of:
a) allele specific oligonucleotide hybridization;
b) size analysis;
c) sequencing;
d) hybridization;
e) 5' nuclease digestion;
f) single-stranded conformation polymorphism;
g) allele specific hybridization;
h) primer specific extension; and
j) oligonucleotide ligation assay.

9. The method of claim 8, wherein the nucleic acid sample is subjected to an amplification step.

10. The method of claim 8, wherein said size analysis is preceded by a restriction enzyme digestion.

11. The method of claim 7, wherein the sepsis therapeutic is a modulator of an IL-1 activity.

12. The method of claim 11, wherein the IL-1 activity is IL-1α.

13. The method of claim 11, wherein the IL- 1 activity is IL-1β.

14. The method of claim 11, wherein the IL-1 activity is IL-1RN.

15. The method of claim 11, wherein the modulator is an IL-1 agonist.

16. The method of claim 11, wherein the modulator is an IL-1 antagonist.

## Patentansprüche

1. Methode zum Bestimmen, ob ein Subjekt für die Entwicklung von und/oder die schnelle Fortentwicklung zu einer Sepsis anfällig ist oder nicht, welche Methode die folgenden Schritte umfasst:
a) Gewinnen einer Nukleinsäure-Probe von dem Subjekt; und
b) Detektieren eines IL-1 Muster 2-Allels in der Probe, wobei das IL-1 Muster 2-Allel ausgewählt ist aus der Gruppe, bestehend aus Allel 1 von IL-1A (+4845) und Allel 2 von IL-1B (-511), wobei der Nachweis eines Muster 2-Allels anzeigt, dass der Patient eine erhöhte Anfälligkeit für die Entwicklung einer Sepsis aufweist.

2. Methode nach Anspruch 1, wobei das IL-1 Muster 2-Allel das Allel 1 von IL-1A (+4845) ist.

3. Methode nach Anspruch 1, wobei der Nachweisschritt ausgewählt ist aus der Gruppe, bestehend aus:
a) Allel-spezifischer Oligonukleotid-Hybridisation;
b) Größenanalyse;
c) Sequenzierung;
d) Hybridisation;
e) 5'-Nuklease-Verdau;
f) einzelsträngigem Konformationspolymorphismus;
g) Allel-spezifischer Hybridisation;
h) Primer-spezifischer Extension; und
j) Oligonukleotid-Ligationsassay.

4. Methode nach Anspruch 1, außerdem umfassend das Amplifizieren der Nukleinsäure-Probe.

5. Methode nach Anspruch 3, wobei der Größenanalyse ein Restriktionsenzym-Verdau vorangeht.

6. Methode nach Anspruch 1, wobei das IL-1 Muster 2-Allel das IL-1B Allel 2 (-511) ist.

7. Methode zum Auswählen eines Therapeutikums für die Behandlung oder Verhütung von Sepsis in einem Subjekt, umfassend:
a) Bestimmen, ob das Subjekt ein IL-1 Muster 2-Allel aufweist, wobei das IL-1 Muster 2-Allel ausgewählt ist aus der Gruppe, bestehend aus Allel 1 von IL-1A (+4845) und Allel 2 von IL-1B (-511); und
b) Auswählen eines entsprechenden Sepsistherapeutikums.

8. Methode nach Anspruch 7, wobei das Detektieren unter Anwendung einer Technik vorgenommen wird, ausgewählt aus der Gruppe, bestehend aus:
a) Allel-spezifischer Oligonukleotid-Hybridisation;
b) Größenanalyse;
c) Sequenzierung;
d) Hybridisation;
e) 5'-Nuklease-Verdau;
f) einzelsträngigem Konformationspolymorphismus;
g) Allel-spezifischer Hybridisation;
h) Primer-spezifischer Extension; und
j) Oligonukleotid-Ligationsassay.

9. Methode nach Anspruch 8, wobei die Nukleinsäure-Probe einem Amplifikationsschritt unterzogen wird.

10. Methode nach Anspruch 8, wobei der Größenanalyse ein Restriktionsenzym-Verdau vorangeht.

11. Methode nach Anspruch 7, wobei das Sepsistherapeutikum ein Modulator einer II-1-Aktivität ist.

12. Methode nach Anspruch 11, wobei die IL-1-Aktivität IL-1α ist.

13. Methode nach Anspruch 11, wobei die IL-1-Aktivität IL-1β ist.

14. Methode nach Anspruch 11, wobei die IL-1-Aktivität IL-1RN ist.

15. Methode nach Anspruch 11, wobei der Modulator ein IL-1-Agonist ist.

16. Methode nach Anspruch 11, wobei der Modulator ein IL-1-Antagonist ist.

## Revendications

1. Procédé pour déterminer si un sujet est sensible ou non au développement d'une septicémie et/ou à une progression rapide en une septicémie, ledit procédé comprenant les étapes :
a) d'obtention d'un échantillon d'acide nucléique du sujet ; et
b) de détection d'un allèle de forme 2 d'IL-1 dans ledit échantillon, l'allèle de forme 2 d'IL-1 étant choisi dans le groupe constitué par un allèle 1 d'IL-lA (+4845) et un allèle 2 d'IL-1B (-511), dans lequel la détection d'un allèle de forme 2 indique que le patient présente une sensibilité accrue au développement d'une septicémie.

2. Procédé selon la revendication 1, dans lequel l'allèle de forme 2 d'IL-1 est un allèle 1 d'IL-lA (+4845).

3. Procédé selon la revendication 1, dans lequel ladite étape de détection est choisie dans le groupe constitué par :
a) l'hybridation à l'aide d'oligonucléotide(s) spécifique(s) d'allèle ;
b) la granulométrie ;
c) le séquençage ;
d) l'hybridation ;
e) la digestion par une 5'-nucléase ;
f) le polymorphisme de conformation simple brin ;
g) l'hybridation spécifique d'un allèle ;
h) l'extension spécifique d'une amorce ; et
j) l'essai par liaison d'oligonucléotide.

4. Procédé selon la revendication 1, comprenant en outre une amplification de l'échantillon d'acide nucléique.

5. Procédé selon la revendication 3, dans lequel ladite granulométrie est précédée d'une digestion par une enzyme de restriction.

6. Procédé selon la revendication 1, dans lequel l'allèle de forme 2 d'IL-1 est un allèle 2 d'IL-1B (-511).

7. Procédé pour sélectionner une thérapie pour le traitement ou la prévention d'une septicémie chez un sujet, comprenant les étampes :
a) de détermination chez le sujet de la présence d'un allèle de forme 2 d'IL-1, l'allèle de forme 2 d'IL-1 étant choisi dans le groupe constitué par un allèle 1 d'IL-lA (+4845) et un allèle 2 d'IL-1B (-511) ; et
b) de sélection d'une thérapie appropriée contre la septicémie.

8. Procédé selon la revendication 7, dans lequel ladite détection est effectuée au moyen d'une technique choisie dans le groupe constitué par :
a) l'hybridation à l'aide d'oligonucléotide(s) spécifique(s) d'allèle ;
b) la granulométrie ;
c) le séquençage ;
d) l'hybridation ;
e) la digestion par une 5'-nucléase ;
f) le polymorphisme de conformation simple brin ;
g) l'hybridation spécifique d'un allèle ;
h) l'extension spécifique d'une amorce ; et
j) l'essai par liaison d'oligonucléotide.

9. Procédé selon la revendication 8, dans lequel l'échantillon d'acide nucléique est soumis à une étape d'amplification.

10. Procédé selon la revendication 8, dans lequel ladite granulométrie est précédée d'une digestion par une enzyme de restriction.

11. Procédé selon la revendication 7, dans lequel la thérapie contre une septicémie utilise un modulateur d'une activité d'IL-1.

12. Procédé selon la revendication 11, dans lequel l'activité d'IL-1 est IL-1α.

13. Procédé selon la revendication 11, dans lequel l'activité d'IL-1 est IL-1β.

14. Procédé selon la revendication 11, dans lequel l'activité d'IL-1 est IL-1RN.

15. Procédé selon la revendication 11, dans lequel le modulateur est un agoniste d'IL-1.

16. Procédé selon la revendication 11, dans lequel le modulateur est un antagoniste d'IL-1.
